(19) Europäisches Patentamt
European Patent Office
Office européen des brevets

(11) Numéro de publication: **0 324 298 B1**

# (12) FASCICULE DE BREVET EUROPEEN

(45) Date de publication de fascicule du brevet: **23.12.92**

(51) Int. Cl.5: **C07D 401/04**, C07D 491/04, A61K 31/47, A61K 31/535, //(C07D491/04,265:00,221:00)

(21) Numéro de dépôt: **88403352.3**

(22) Date de dépôt: **28.12.88**

Le dossier contient des informations techniques présentées postérieurement au dépôt de la demande et ne figurant pas dans le présent fascicule.

(54) **Dérivés des acides 7-(1-azétidinyl)-1,4-dihydro-4-oxoquinoléine-3-carboxyliques, leur préparation et leur application en tant que médicaments.**

(30) Priorité: **29.12.87 FR 8718289**
**20.07.88 FR 8809816**

(43) Date de publication de la demande:
**19.07.89 Bulletin 89/29**

(45) Mention de la délivrance du brevet:
**23.12.92 Bulletin 92/52**

(84) Etats contractants désignés:
**AT BE CH DE FR GB GR IT LI LU NL SE**

(56) Documents cités:
**EP-A- 0 047 005**
**EP-A- 0 106 489**
**EP-A- 0 160 284**
**EP-A- 0 241 206**
**US-A- 4 617 308**

**CHEMICAL & PHARMACEUTICAL BULLETIN, vol. 32, no. 12, décembre 1984, pages 4907-4913; I. HAYAKAWA et al.: "Synthesis and antibacterial activities of substituted 7-oxo-2,3-dihydro-7H-pyrido[1,2,3-de][1,4]benzoxazine-6-carboxylic acids"**

**CHEMICAL ABSTRACTS, vol. 109, no. 23, 5 décembre 1988, page 664, résumé no. 211034w, Columbus, Ohio, US; & JP - A - 63 60 990**

(73) Titulaire: **LABORATORIOS DEL DR. ESTEVE, S.A.**
**Av. Mare de Deu de Montserrat, 221**
**E-08026 Barcelona(ES)**

(72) Inventeur: **Corominas, Juan Pares**
**Padilla , 349, 30 3a**
**E-08025 Barcelona(ES)**
Inventeur: **Constansa, Jordi Frigola**
**Avenida Diagonal, 299 at. 1a**
**E-08013 Barcelona(ES)**
Inventeur: **Colombo Pinol, Augusto**
**Avenida chile, 36, 40 1a**
**E-08032 Barcelona(ES)**

(74) Mandataire: **Ahner, Francis et al**
**CABINET REGIMBEAU, 26, avenue Kléber**
**F-75116 Paris(FR)**

EP 0 324 298 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à de nouveaux dérivés des acides 1,4-dihydro-4-oxoquinoléine-3-carboxyliques substitués en position 7 par le radical 1-azétidinyle substitué en position 2 et/ou en position 3.

Les azétidines liées à la position 7 des acides 1,4-dihydro-4-oxo-quinoléine-3-carboxyliques ont été très peu étudiées. Autant que l'on sache il n'existe qu'un petit nombre de publications dans la littérature scientifique qui se rapportent à ce genre de composés. Dans les brevets (Japan Kokai Tokkyo Koho JP 58/72589 (83/72589), et Eur. Pat. Appl. EP-A-0047005, EP-A-106489 et EP-A-153163) on décrit l'acide 1-éthyl-7-(3-(éthylamino)méthyl-1-azétidinyl)-6,8-difluoro-1,4-dihydro-4-oxo-3-quinoléincarboxylique, l'acide 9-fluoro-2,3-dihydro-10-(3-hydroxy-1-azétidinyl)-3-méthyl-7-oxo-7H-pirido [1,2,3-de]-1,4-benzoxazine-6-carboxylique, et l'acide 9-fluoro-2,3-dihydro-10-(3-hydroxméthyl-1-azétidinyl)-3-méthyl-7-oxo-7-H-pirido [1,2,3-de]-1,4-benzoxazine-6-carboxylique.

L'invention concerne des composés hétérocycliques représentés par la formule (I) ci-après, ainsi que les sels thérapeutiquement acceptables de ces composés :

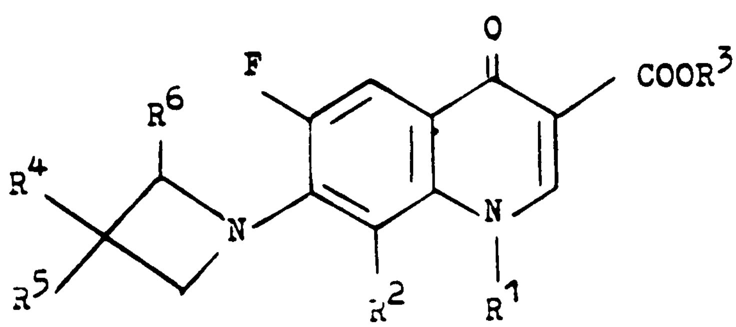

(I)

où $R^1$ représente un radical alkyle en $C_1$ à $C_4$, un radical halogénoalkyle en $C_1$ à $C_4$, un radical cyclopropyle, un radical aminoalkyle en $C_1$ à $C_4$, un radical phényle ou un radical pnényle substitué par un ou plusieurs atome(s) de fluor,
$R^2$ représente un atome d'hydrogène, un atome d'halogène, ou bien $R^1$ et $R^2$ peuvent former ensemble une liaison représentée par un groupe X,
$R^3$ représente un atome d'hydrogène ou un radical alkyle inférieur en $C_1$ à $C_4$,
$R^4$ et $R^5$ et $R^6$ représentent indépendamment un atome d'hydrogène, un radical alkyle en $C_1$ à $C_4$, un radical hydroxyle, un radical amino, un radical aminoalkyle en $C_1$ à $C_4$, un radical alkyl en $C_1$ à $C_4$ amino, un radical dialkyl en $C_1$ à $C_4$ amino, un radical alkyl en $C_1$ à $C_4$ aminoalkyle en $C_1$ à $C_4$, un radical alcoxy en $C_1$ à $C_4$, un radical mésyloxy, un radical hydroxy-alkyle en $C_1$ à $C_4$, un radical cyano, un radical acylaminoalkyle en $C_1$ à $C_4$, un radical carboxylique, un radical carboxamido, un radical carboxyalkyle en $C_1$ à $C_4$, un atome d'halogène, un radical alkyl en $C_1$ à $C_4$ carboxylique, par exemple acétoxy, un radical acétamido ou un radical acétamidoalkyle en $C_1$ à $C_4$, le groupe alkyle en $C_1$ à $C_4$ libre terminal pouvant être fluoré dans ces deux derniers radicaux et l'atome d'azote dans le radical acétamidoalkyle en $C_1$ à $C_4$ pouvant être substitué par un groupe alkyle en $C_1$ à $C_4$.
X représente $-CH_2-CH_2-CHR^7-$, ou

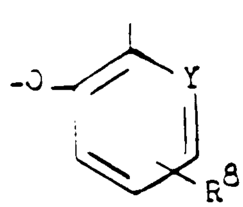

où $R^7$ représente un atome d'hydrogène ou un radical alkyle inférieur en $C_1$ à $C_4$
$R^8$ représente un atome d'hydrogène ou un atome d'halogene, et Y représente CH ou N,
à l'exception toutefois des composés de formule (I), dans laquelle :
$R^1$ représente un radical éthyle

$R^2$ représente un atome de fluor
$R^3$, $R^4$ et $R^6$ représentent un atome d'hydrogène, et
$R^5$ représente un radical éthylaminométhyle ($CH_3CH_2NHCH_2$).

Certains composés selon l'invention sont plus précisément représentés par la formule générale (Ia)

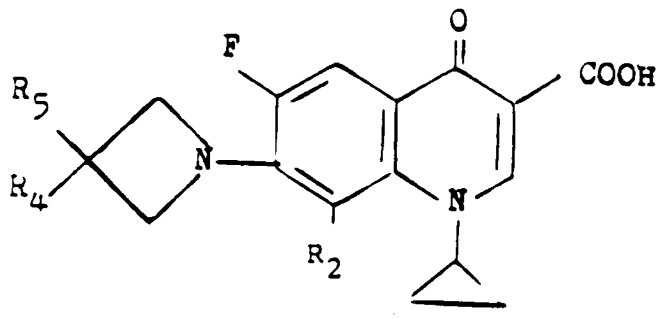

(Ia)

dans laquelle $R_2$, $R_4$ et $R_5$ ont la même signification que précédemment.

L'invention concerne également une composition pharmaceutique contenant un composé de formule (I) où un de ses sels pharmaceutiquement acceptable en une quantité antimicrobienne efficace.

De plus, l'invention concerne des procédés pour préparer les composés de formule (I) et leurs sels pharmaceutiquement acceptables.

Dans l'ensemble de la description le terme alkyle inférieur désignera des radicaux hydrocarbonés linéaires ou ramifiés comprenant de 1 à 4 atomes de carbone.

On peut préparer les composés de l'invention représentés par la formule (I) selon divers procédés. Par exemple, un procédé consiste à faire réagir un composé hétérocyclique de formule (II)

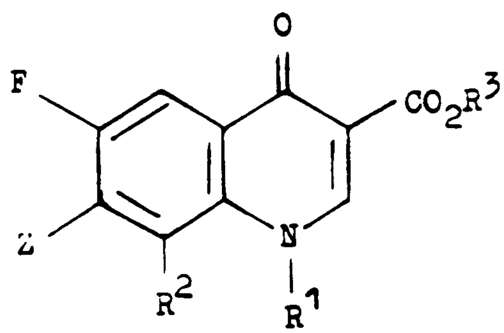

(II)

où $R^1$, $R^2$ et $R^3$ ont la même signification que précédemment, et Z représente un atome d'halogène ; avec un composé représenté par la formule (III)

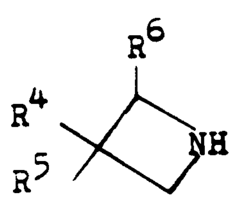

(III)

où $R^4$ et $R^5$ et $R^6$ ont la même signification que précédemment.

On peut effectuer la réaction dans un grand nombre de solvants. On peut citer comme exemples, des alcools inférieurs tels que l'éthanol, l'isopropanol, etc., des éthers tels que le tétrahydrofuranne, le dioxanne, le diglyme, etc, des nitriles tels que l'acétonitrile, la pyridine, le diméthylsulfoxyde, le diméthylformamide et l'hexaméthylphosphorotriamide.

On peut effectuer la réaction ci-dessus en présence d'un accepteur d'acide, en une quantité au moins approximativement comprise entre 1 et 2 moles par mole de composé de formule (II). On peut citer comme exemples d'accepteurs d'acide appropriés, des hydroxydes de métal alcalin, des carbonates minéraux, et des amines tertiaires telles que la triéthylamine.

On peut effectuer la réaction ci-dessus sous pression, c'est-à-dire à une pression d'environ 1 à 15 kg/cm$^2$, et à une température d'environ 50 à 250 °C pendant une durée d'environ 2 à 24 heures.

Les composés hétérocycliques de formule (II) que l'on peut utiliser comme matières de départ pour préparer les composés de l'invention représentés par la formule (I) sont des composés connus, comme décrit par exemple dans H. Koga, A. Itoh, S. Murayama, S. Suzue et T. Irikura, J. Med. Chem., 1980, **23**, 1358.

D'autre part, les composés de formule (III) qui constituent d'autres matières de départ pour préparer les composés de l'invention représentés par la formule (I) sont connus, ou sont synthétisés comme décrit par exemple dans divers articles (A.G. Anderson et R. Lok, J. Org. Chem., 1972, **37**, 3953, R.H. Higgins et N.H. Cromwell, J. Heterocycl. Chem., 1971, 8, 1059).

On peut préparer aussi les composés de l'invention représentés par la formule (I) par un procédé qui consiste à faire réagir un composé hétérocyclique de formule (II), où $R^1$, $R^2$ et $R^3$ ont la même signification que précédemment, et Z représente un radical amino ; avec un composé représenté par la formule (IV)

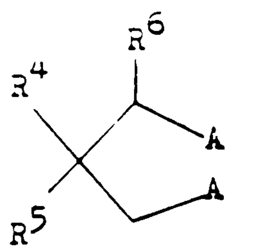

(IV)

où $R^4$ et $R^5$ et $R^6$ ont la même signification que précédemment, et A représente un atome d'halogène, un radical hydroxyle, un radical alkylsulfonyloxy inférieur ou un radical arylsulfonyloxy.

On peut effectuer la réaction dans des solvants tels que des alcools inférieurs ou des solvants dipolaires aprotiques tels que le diméthylsulfoxyde, le diméthylformamide et l'hexaméthylphosphorotriamide.

On peut effectuer la réaction ci-dessus en présence d'un accepteur d'acide approprié, tel que des hydroxydes de métal alcalin, des carbonates minéraux, et des amines tertiaires telles que la pyridine ou la triéthylamine.

On peut effectuer la réaction ci-dessus à pression atmosphérique ou à une pression d'environ 1 à 15 kg/cm$^2$, et à une température d'environ 10 à 50 °C pendant une durée d'environ 1 à 5 jours et après à une température d'environ 50 à 150 °C pendant une durée d'environ 8 à 72 heures.

Les composés hétérocycliques de formule (II) dans lesquels Z représente un radical amino, que l'on peut utiliser comme matières de départ pour préparer les composés de l'invention représentés par la formule (I), sont des composés connus, comme décrit par exemple dans le brevet EP 0 134 165 et dans deux publications (T. Uno, M. Takamatsu, Y.Inone, Y; Kawahata, K. Iuchi, G. Tsukamoto, J. Med. Chem., 1987, **30**, 2163 ; et dans H. Koga, A. Itoh, S. Murayama, S. Suzue et T. Irikura, J. Med. Chem., 1980,

1358). D'autre part, les composés de formule (IV) qui constituent d'autres matières de départ, sont des produits commerciaux.

Parmi les composés représentés par la formule (I) ceux où $R^3$ représente un atome d'hydrogène et/ou $R^4$ ou $R^5$ ou $R^6$ représentent un radical amino, un radical aminoalkyle, un radical alkylamino, un radical alkylaminoalkyle, peuvent être préparés par hydrolyse des composés représentés par la formule (I) ceux où $R^3$ représente un radical alkyle inférieur et/ou $R^4$ ou $R^5$ ou $R^6$ représentent un radical acylamino, un radical acylaminoalkyle, un radical alkylacylamino ou un radical alkylacylaminoalkyle.

On peut effectuer la réaction d'hydrolyse selon des procédés classiques par exemple en présence d'un catalyseur classique, tel qu'un composé basique comme l'hydroxyde de sodium, l'hydroxyde de potassium et similaires, un acide minéral comme l'acide sulfurique, l'acide chlorhydrique, ou un acide organique comme un acide sulfonique aromatique et similaires.

De façon générale, on peut effectuer la réaction dans un solvant classique tel que l'eau, des alcools, le dioxanne, l'acétone, ou un mélange d'entre eux. La température de réaction est généralement comprise entre la température du laboratoire et 150 ° C, pendant une durée d'environ 2 à 24 heures.

Dans les exemples suivants on indiquera la préparation de nouveaux dérivés selon l'invention. On décrira également quelques formes d'emploi typiques pour les différents domaines d'application.

Les exemples ci-après, donnés à simple titre d'illustration, ne doivent cependant en aucune façon limiter l'étendue de l'invention.

## EXEMPLE 1 :

### Méthode A

Préparation du 1-cyclopropyl-6,8-difluoro-7-(3 hydroxy-1-azétidinyl)-1,4-dihydro-4-oxo-3-quinoléincarboxylate d'éthyle

On chauffe pendant 4 heures à 80 ° C une solution de 1,22 g (3,92 mmoles) de 1-cyclopropyl-6,7,8-trifluoro-1,4-dihydro-4-oxo-3-quinoléincarboxylate d'éthyle, 0,86 g (7,85 mmoles) de chlorhydrate de 3-hydroxyazétidine, 2 g (19,8 mmoles) de triéthylamine et 20 ml de diméthylsulfoxyde (DMSO). On laisse refroidir et on additionne sur $H_2O$/glace, en obtenant un précipité que l'on filtre et on lave avec de l'eau. On sèche le solide sous vide et on obtient 1,40 g (97%) de 1-cyclopropyl-6,8-difluoro-7-(3-hydroxy-1-azétidinyl)-1,4-dihydro-4-oxo-3-quinoléincarboxylate d'éthyle de point de fusion 260 ° -270 ° C.
Données spectroscopiques :
$^1H$ RMN, $\delta$, [DMSO-$d_6$] : 1,08 (d, 4H, J = 5 Hz); 1,26 (t, 3H, J = 7 Hz); 3,60-4,80 (m, 6H); 5,66 (d, 1H, J = 4 Hz); 7,52 (d, 1H, J = 13,5 Hz); 8,32 (s, 1H). IR (KBr) : 3300; 1725; 1615 $cm^{-1}$.

### Méthode B

Préparation du 1-cyclopropyl-6,8-difluoro-7-(3-hydroxy-1-azétidinyl)-1,4-didhyro-4-oxo-3-quinoléincarboxylate d'éthyle

On laisse sous agitation et protégée de la lumière à température ambiante pendant 3 jours une solution de 0,8 g (2,60 mmoles) de 7-amino-1-cyclopropyl-6,8-difluoro-1,4-dihydro-4-oxo-3-quinoléincarboxylate d'éthyle, 0,33 g (2,60 mmoles) de 1,3-dichloro-2-propanol et de 25 ml de pyridine ; puis on laisse à reflux pendant 3 jours de plus. On concentre presqu'à sec, on verse sur de l'eau et on obtient un précipité que l'on filtre et on lave à l'eau. On sèche le solide sous vide et on obtient 0,52 g (55 %) de 1-cyclopropyl-6,8-difluoro-7-(3-hydroxy-1-azétidinyl)-1,4-dihydro-4-oxo-3- quinoléincarboxylate d'éthyle. Point de fusion et données spectroscopiques identiques à ceux du dérivé obtenu selon la méthode A.

## EXEMPLE 2 :

### Méthode C

Préparation de l'acide 1-cyclopropyl-6,8-difluoro-7-(3-hydroxy-1-azétidinyl)-1,4-dihydro-4-oxo-3-quinoléincarboxylique.

On laisse à reflux pendant 1,5 heure une solution de 0,4 g (1,10 mmoles) de 1-cyclopropyl-6,8-difluoro-7-(3-hydroxy-1-azétidinyl)-1,4-dihydro-4-oxo-3-quinoléincarboxylate d'éthyle, 2 ml d'éthanol et 10 ml de soude 0,5 N. On laisse refroidir, on dilue avec de l'eau, on ajuste à pH 5 et on obtient un précipité que l'on filtre et on lave à l'eau. On sèche le solide sous vide et on obtient 0,37 g (100 %) de l'acide 1-cyclopropyl-6,8-difluoro-7-(3-hydroxy-1-azétidinyl)-1,4-dihydro-4-oxo-3-quinoléincarboxylique de point de fusion 286-288° C.
Données spectroscopiques :
$^1$H RMN, δ, [DMSO-$d_6$, TFA] : 1,13 (m, 4H); 4,10 (m, 3H); 4,55 (m, 3H); 7,75 (d, 1H, J = 13 Hz); 8,55 (s, 1H). IR (KBr) : 3400; 1700; 1625 $cm^{-1}$.

Méthode D

Préparation de l'acide 1-cyclopropyl-6,8-difluoro-7-(3-hydroxy-1-azétidinyl)-1,4-dihydro-4-oxo-3-quinoléincarboxylique

On chauffe à 80° C pendant 4 heures une solution de 0,9 g (3,2 mmoles) d'acide 1-cyclopropyl-6,7,8-trifluoro-1,4-dihydro-4-oxo-3-quinoléincarboxylique, 0,7 g (6,4 mmoles) de chlorhydrate de 3-hydroxy-azétidine, 1,6 g (16,0 mmoles) de triéthylamine et 15 ml de DMSO. On laisse refroidir, on additionne sur un mélange eau/glace, on ajuste à pH 5 et on obtient un précipité que l'on filtre et on lave à l'eau. On sèche le solide sous vide et on obtient 0,86 g (80 %) d'acide 1-cyclopropyl-6,8-difluoro-7-(3 hydroxy-1-azétidinyl)-1,4-dihydro-4-oxo-3-quinoléincarboxylique, de point de fusion 286-288° C. Données spectroscopiques identiques à celles de la méthode C.

EXEMPLE 3 :

Préparation du 1-cyclopropyl-6,8-difluoro-7-(3-mésyloxy-1-azétidinyl)-1,4-dihydro-4-oxo-3-quinoléincarboxylate d'éthyle

On additionne lentement 6,3 g (55,0 mmoles) de chlorure de mésyle à une solution refroidie à 0° C de 1,0 g (2,75 mmoles) de 1-cyclopropyl-6,8-difluoro-7-(3-hydroxy-1-azétidinyl)-1,4-dihydro-4-oxo-3-quinoléincarboxylate d'éthyle dans 50 ml de pyridine et on maintient la réaction à 0° C pendant 3 heures. On additionne sur un mélange eau/glace et on obtient un précipité que l'on filtre et on lave à l'eau. On sèche le solide sous vide et on obtient 0,90 g (73 %) de 1-cyclopropyl-6,8-difluoro-7-(3-mésyloxy-1-azétidinyl-1,4-dihydro-4-oxo-3-quinoléincarboxylate d'éthyle de point de fusion 191-193° C.
Données spectroscopiques :
$^1$H RMN, δ, [$CDCl_3$] : 1,11 (é, 4H); 1,38 (t, 3H, J = 7Hz); 3,08 (s, 3H); 3,80 (m, 1H); 4,36 (q, 2H, J = 7 Hz); 4,53 (m, 2H); 4,70 (m, 2H); 5,36 (m, 1H); 7,83 (dd, 1H, J = 13 Hz, J' = 1 Hz); 8,45 (s, 1H). IR 'KBr) : 1720; 1615; 1475; 1340; 1165 $cm^{-1}$.

EXEMPLE 4 :

Préparation du 7-(3-acétamidométhyl-1-azétidinyl)-1-cyclopropyl-6,8-difluoro-1,4-dihydro-4-oxo-3-quinoléincarboxylate d'éthyle.

On chauffe à 80° C pendant 4 heures une solution de 1,0 g (3,2 mmoles) de 1-cyclopropyl-6,7,8-trifluoro-1,4-dihydro-4-oxo-3-quinoléincarboxylate d'éthyle, 1,05 g (6,4 mmoles) de chlorhydrate de 3-acétamido-méthylazétidine, 1,6 g (16 mmoles) de triéthylamine et 20 ml de DMSO. On laisse refroidir, on verse sur un mélange eau/glace et on obtient un précipité que l'on filtre et on lave à l'eau. On sèche le solide sous vide et on obtient 0,93 g (69 %) de 7-(3-acétamidométhyl-1-azétidinyl)-1-cyclopropyl-6,8-difluoro-1,4-dihydro-4-oxo-3-quinoléincarboxylate d'éthyle de point de fusion 170-190° C.
Données spectroscopiques :
$^1$H RMN, δ, [$CDCl_3$] : 1,11 (m, 4H); 1,37 (t, 3H, J = 7 Hz); 2,04 (s, 3H); 2,97 (m, 1H); 3,4-4,7 (m, 9H); 6,64 (m, 1H); 7,67 (d, 1H, J = 13 Hz); 8,44 (s, 1H). IR(KBr) : 3300; 1720; 1650; 1615; 1545 $cm^{-1}$.

EXEMPLE 5 :

Préparation de l'acide 1-cyclopropyl-6,8-difluoro-7-(1-azétidinyl-3-carboxylique)-1,4-dihydro-4-oxo-3-quinoléincarboxylique.

On chauffe à 100 ° C pendant 24 heures un mélange de 0,3 g (1 mmole)d'acide 1-cyclopropyl-6,7,8-trifluoro-1,4-dihydro-4-oxo-3-quinoléincarboxylique, 0,2 g (2 mmoles) d'acide azétidin-3-carboxylique, 0,5 g (5 mmoles) de triéthylamine et 5 ml de DMSO.

On laisse refroidir, on additionne sur un mélange eau/glace et on filtre et recristallise du DMF/$H_2O$ (15:2) et on obtient 0,11 g (28 %) de l'acide 1-cyclopropyl-6,8-fluoro-7-(1-azétidinyl-3-carboxylique)-1,4-dihydro-4-oxo-3-quinoléincarboxylique de point de fusion 251-5 ° C.

Données spectroscopiques :

$^1H$ RMN, δ, [DMSO-$d_6$] : 1,2 (m, 4H); 3,55 (m, 1H); 3,95 (m, 1H); 4,52 (m, 5H); 7,65 (d, 1H, J = 12 Hz); 8,55 (s, 1H).

IR(KBr) : 2920, 1725, 1630, 1460 $cm^{-1}$.

EXEMPLE 6 :

Préparation de l'acide 1-cyclopropyl-6,8-difluoro-7-(1-azétidinyl-3-carboxamide)-1,4-dihydro-4-oxo-3-quinoléincarboxylique.

On chauffe à 100 ° C pendant 2 heures un mélange de 0,57 g (2 mmoles) d'acide 1-cyclopropyl-6,7,8-trifluoro-1,4-dihydro-4-oxo-3-quinoléincarboxylique, 0,35 g (2,6 mmoles) de l'hydrochlorure d'azétidine-3-carboxamide, et 0,6 g (6 mmoles) de triéthylamine dans 5 ml de DMSO.

On laisse refroidir, on additionne sur un mélange eau/acide acétique et on filtre et lave à l'eau, et on obtient 0,62 g (66 %) de l'acide 1-cyclopropyl-6,8-difluoro-7-(1-azétidinyl-3-carboxamide)-1,4-dihydro-4-oxo-3-quinoléincarboxylique de point de fusion 295-8 ° C.

Données spectroscopiques :

$^1H$ RMN, δ, [DMSO-6d, TFA]; 1,15 (m, 4H); 3,55 (m, 1H); 4,05 (m, 1H); 4,45 (m, 4H); 7,1 (s, 1H); 7,55 (m, 2H); 8,6 (s, 1H).

IR(KBr) : 3390, 3190, 1740, 1665,1640, 1450 $cm^{-1}$.

EXEMPLE 7 :

Préparation de l'acide 1-cyclopropyl-6,8-difluoro-7-(1-azétidinyl-3-cyano)-1,4-dihydro-4-oxo-3-quinoléincarboxylique

On chauffe à reflux pendant 24 heures une solution de 0,57 g (1,5 mmoles) de l'acide 1-cyclopropyl-6,8-difluoro-7-(1-azétidinyl-3-carboxamide)-1,4-dihydro-4-oxo-3-quinoléincarboxylique (exemple 6) dans 12 ml d'anhydride acétique. On refroidit, on filtre et on lave à l'eau et à l'éthanol. On obtient ainsi 0,15 g (27 %) de l'acide 1-cyclopropyl-6,8-difluoro-7-(1-azétidinyl-3-cyano)-1,4-dihydro-4-oxo-3-quinoléincarboxylique de point de fusion > 325 ° C.

Données spectroscopiques :

$^1H$ RMN, δ, [DMSO-6d, TFA] : 1,20 (m, 4H); 3,95 (m, 1H); 4,6 (m, 5H); 7,75 (d, J = 12 Hz, 1 H); 8,6 (s, 1H).

IR(KBr) : 2250, 1735, 1635, 1650 $cm^{-1}$.

EXEMPLE 8 :

Préparation de l'acide 1-cyclopropyl-6,8-difluoro-7-(1-azétidinyl-3-méthyl-3-hydroxy)-1,4-dihydro-4-oxo-3-quinoléincarboxylique

On chauffe à reflux pendant 10 heures un mélange de 0,9 g (2,9 mmoles) de 1-cyclopropyl-6, 7, 8-trifluoro-1,4-dihydro-4-oxo-3-quinoléincarboxylate d'éthyle, 0,54 g (4,3 mmoles) de l'hydrochlorure de 3-hydroxy-3-méthylazétidine, 1 g (10,8 mmoles) de triéthylamine et 10 ml de pyridine. On refroidit et on dilue avec de l'eau, on filtre et lave. On obtient ainsi 0,95 g (89 %) de 1-cyclopropyl-6,8-difluoro-7-(1-azétidinyl-3-méthyl-3-hydroxy)-1,4-dihydro-4-oxo-3-quinoléincarboxylate d'éthyle qui est ensuite hydrolysé en chauffant un mélange de 0,38 g (1 mmole)de cet ester avec 1,5 ml d'éthanol, et 8 ml de soude 0,5 N à reflux pendant 3 heures. On refroidit, on filtre et on acidifie avec de l'acide acétique.

On filtre et lave à l'eau, et on obtient 0,34 g (97 %) de l'acide 1-cyclopropyl-6,8-difluoro-7-(1-acétidinyl-3-méthyl-4-hydroxy)-1,4-dihydro-4-oxo-3-quinoléincarboxylique avec un point de fusion 290-4 ° C.

Données spectroscopiques :

$^1H$ RMN, δ, [DMSO-6d, TFA] : 1,16 (d, J = $7H_z$, 4H); 1,48 (s, 3H); 4,05 (m, 1H); 4,26 (m, 4H); 7,66 (dd J = 13 Hz, J = 2 Hz, 1H); 8,56 (s, 1H).

IR(KBr) : 3450, 1725, 1630, 1530, 1460 $cm^{-1}$.

EXEMPLE 9 :

Préparation de l'acide 7-(3-trifluoroacétamido-méthyl-1-azétidinyl)-1-cyclopropyl-6,8-difluoro-1,4-dihydro-4-oxo-3-quinoléincarboxylique

On chauffe à reflux pendant 3 heures une solution de 0,8 g (2,8 mmoles) de l'acide 1-cyclopropyl-6,7,8-trifluoro-1,4-dihydro-4-oxo-3-quinoléincarboxylique, 0,92 g (4,2 mmoles) du chlorhydrate de 3-trifluoroacétamidométhylazétidine, 8 ml de pyridine et 1,7 g de triéthylamine. On évapore sous vide, on dilue avec de l'eau et on filtre. On obtient ainsi 1,12 g (88,9 % de l'acide 7-(3-trifluoroacétamidométhyl-1-azétidinyl)-1-cyclopropyl-6,8-difluoro-1,4-dihydro-4-oxo-3-quinoléincarboxylique, de point de fusion 145-150° C.
Données spectroscopiques :
$^1$H RMN, δ, [DMSO-6d, TFA] : 1,10 (m, 4H); 3,0 (m, 1H); 3,50 (m, 2H); 4,20 (m, 3H); 4,50 (m, 2H); 7,65 (d J = 13Hz1H); 8,45 (s, 1H).
IR(KBr) : 3300, 1725, 1630, 1460 $cm^{-1}$.

EXEMPLE 10 :

Préparation de l'acide 7-(3-aminométhyl-1-azétidinyl)-1-cyclopropyl-6,8-difluoro-1,4-dihydro-4-oxo-3-quinoléincarboxylique.

On maintient à 80° C pendant 3 heures une solution de 0,8 g (1,6 mmoles) d'acide 7-(3-trifluoroacétamidométhyl-1-azétidinyl)-1-cyclopropyl-6,8-difluoro-1,4-dihydro-4-oxo-3-quinoléincarboxylique (exemple 9), dans 30 ml de soude 1N, on refroidit et on acidifie avec de l'acide acétique, on filtre, on lave et on obtient 0,41 g (65 %) de l'acide 7-(3-aminométhyl-1-azétidinyl)-1- cyclopropyl-6,8-difluoro-1,4-dihydro-4-oxo-3-quinoléincarboxylique avec point de fusion 190-195° C.
Données spectroscopiques :
$^1$H RMN, δ, [DMSO-6d, TFA] : 1,16 (m, 4H); 3,0 (m, 2H); 4,25 (m, 5H); 7,71 (m, 3H); 8,55 (s, 1H).
IR(KBr) : 3500, 1730, 1680, 1630 $cm^{-1}$.

EXEMPLE 11 :

Préparation de l'acide 1-cyclopropyl-6,8-difluoro-7-(3-mésyloxy-1-azétidinyl)-1,4-dihydro-4-oxo-3-quinoléincarboxylique

On maintient à reflux pendant 1 heure une solution de 0,2 g (0,4 mmole) du 1-cyclopropyl-6,8-difluoro-7-(3-mésyloxy-1-azétidinyl)-1,4-dihydro-4-oxo-3-quinoléincarboxylate d'éthyle (exemple 3) dans 6 ml de soude 0,5 N et 1 ml d'éthanol. On évapore sous vide et on additionne de l'acide acétique, on filtre, on lave et on obtient 0,18 g (96 %) de l'acide 1-cyclopropyl-6,8-difluoro-7-(3-mésyloxy-1-azétidinyl)-1,4-dihydro-4-oxo-3-quinoléincarboxylique de de point de fusion 240-4° C.
Données spectroscopiques :
$^1$H RMN, δ, [DMSO-6d, TFA] : 1,19 (m, 4H); 3,3 (s, 3H); 4,06 (m, 2H); 4,54 (m, 2H); 4,77 (m, 2H); 5,44 (m, 1H); 7,68 (d, J = 14Hz 1H); 8,57 (s, 1H).

EXEMPLE 12 :

Préparation de l'acide 7-[3-(N'-éthyl-N'-trifluoroacétamidométhyl)-1-azétidinyl]-6,8-difluoro-1,4-dihydro-1-cyclopropyl-4-oxo-3-quinoléincarboxylique

On chauffe à reflux pendant 2 heures une solution de 1,0 g (3,5 mmoles) de l'acide 1-cyclopropyl-6,7-8-trifluoro-1,4-dihydro-4-oxo-3-quinoléincarboxylique, 1,4 g (5,7 mmoles) du chlorhydrate de 3-(N'-éthyl-N'-trifluoroacétamidométhylazétidine, 9 ml de pyridine et 2,9 g (28,5 mmoles) de triétylamine. On évapore sous vide et on dilue avec une solution d'éthanol dans l'eau (1 : 1), on filtre et on lave. On obtient 1,3 g (78 %) de l'acide 7-[3-(N'-éthyl-N'-trifluoroacétamidométhyl)-1-azétidinyl]-6,8-difluoro-1,4-dihydro-1-cyclopropyl-4-oxo-3-quinoléincarboxylique de point de fusion 208-12° C.
Données spectroscopiques :
$^1$H RMN, δ, [DMSO-6d, TFA] : 1,15 (m, 7H); 3,0 (m, 1H); 3,35 (m, 2H); 3,72 (m, 2H); 4,1 (m, 3H); 4,45 (m,

2H); 7,6 (d, J = 13Hz 1H); 6,55 (s, 1H).
IR(KBr) : 1729, 1688, 1466, 1326 $cm^{-1}$.

EXEMPLE 13 :

Préparation de l'acide 7-(3- éthylaminométhyl-1-azétidinyl)-6,8-difluoro-1,4-dihydro-1-cyclopropyl-4-oxo-3-quinoléincarboxylique

On maintient à reflux pendant 3 heures une solution de 0,7 g (1,5 mmoles) de l'acide 7-[3-(N'éthyl-N'-trifluoroacétamidométhyl)-1-azétidinyl] 6,8-difluoro-1,4-dihydro-1-cyclopropyl-4-oxo-3-quinoléincarboxylique (exemple 12), dans 9 ml de soude 1N et 3 ml d'éthanol. On refroidit, on additionne de l'acide acétique, on filtre et on lave à l'éthanol froid. On obtient 0,37 g (66 %) de l'acide 7 (3-éthylaminométhyl-1-azétidinyl)-6,8-difluoro-1,4-dihydro-1-cyclopropyl-4-oxo-3-quinoléincarboxylique avec point de fusion 237-42° C.
Données spectroscopiques :
$^{1}H$ RMN, δ, [DMSO-6d, TFA] : 1,2 (m, 7H); 2,6 (m, 1H); 3,0 (m, 2H); 3,25 (m, 2H); 4,05 (m, 1H); 4,25 (m, 2H); 4,5 (m, 2H); 7,6 (d, J = 13Hz, 1H); 8,5 (s, 1H).
IR(KBr) : 3300, 1624, 1474, 1323 $cm^{-1}$.

EXEMPLE 14 :

Préparation de l'acide 1-cyclopropyl-6,8-difluoro-1,4-dihydro-7-(1-azétidinyl)-4-oxo-3-quinoléincarboxylique

On maintient à 110-120° C pendant 2 heures un mélange de 0,6 g (2,1 mmoles de l'acide 1-cyclopropyl-1,4-dihydro-6,7,8-trifluoro-4-oxo-3-quinoléincarboxylique avec 0,25 g (4,4 mmoles) d'azétidine, 8 ml de pyridine et 1 ml de triéthylamine, dans un récipient clos. On refroidit, on évapore sous vide et on filtre et lave et on obtient 0,6 g (88 %) de l'acide 1-cyclopropyl-6,8-difluoro-1,4-dihydro-7-(1-azétidinyl)-4-oxo-3-quinoléincarboxylique avec point de fusion 289-93° C.
Données spectroscopiques :
$^{1}H$ RMN, δ, [DMSO-6d] : 1,15 (m, 4H); 2,50 (m, 2H); 4,07 (m, 1H); 4,45 (m, 4H); 7,70 (d, J = 13Hz, 1H); 8,58 (s, 1H).
IR(KBr) : 1724, 1629, 1460 $cm^{-1}$.

EXEMPLE 15 :

Préparation de l'acide 1-cyclopropyl-6,8-difluoro-7-(3-méthyl-3-trifluoroacétamido-1-azétidinyl)-4-oxo-3-quinoléincarboxylique

On maintient à reflux pendant 3 heures une solution de 1 g (3,5 mmoles) de l'acide 1-cyclopropyl-6,7,8-trifluoro-4-oxo-3-quinoléincarboxylique, 1,15 g (5,3 mmoles) de l'hydrochlorure de 3-méthyl-3-trifluoroacétamido azétidine, 2 ml de triéthylamine dans 10 ml de pyridine.

On évapore sous vide, on additionne de l'eau et on acidifie avec de l'acide acétique et on filtre et lave à l'eau et à l'éthanol froid. On obtient 1,15 g (73 %) de l'acide 1-cyclopropyl-6,8- difluoro-1,4-dihydro-7-(3-méthyl-3-trifluoroacétamido-1-azétidinyl)-4-oxo-3-quinoléincarboxylique avec point de fusion 208-213° C.
Données spectroscopiques :
$^{1}H$ RMN, δ, [DMSO-$d_6$-TFA] : 1,1 (élargie, 4H); 1,5 (s, 3H); 4,0 (m, 1H); 4,2 (m, 2H); 4,5 (m, 2H); 7,5 (m, 1H); 8,5 (s, 1H); 9,8 (s, 1H).
IR(KBr) : 3320, 1725, 1628, 1465 $cm^{-1}$.

EXEMPLE 16 :

Méthode E : Préparation de l'acide 1-cyclopropyl-6,8-difluoro-1,4-dihydro-7-(3-méthyl-3-amino-1-azétidinyl)-4-oxo-3- quinoléincarboxylique

On maintient à reflux une solution de 0,8 g (1,8 mmoles) de l'acide -1-cyclopropyl-6,8-difluoro-1,4-dihydro-7-(-3-méthyl-3-trifluoroacétamido-1-azétidinyl)-4-oxo-3-quinoléincarboxylique (exemple 15) dans un mélange de 10 ml de soude 1N et 2 ml d'éthanol, pendant 3 heures. On évapore sous vide et on additionne de l'acide acétique. On filtre et on lave à l'eau et à l'éthanol. On obtient 0,35 g (55%) de l'acide 1-cyclopropyl-6,8-difluoro-1,4-dihydro-7-(3-méthyl-3-amino-1-azétidinyl)-4-oxo-3-quinoléincarboxylique de

point de fusion 298-300 ° C.
Données spectroscopiques
$^{1}$H RMN, δ, [DMSO-$d_6$, TFA]: 1,18 (d, 4H, J = 6,2Hz); 1,64 (s, 3H); 4,05 (m, 1H); 4,42 (m, 4H); 7,74 (dd 1H, J = 12,5Hz, J' = 1,7Hz); 8,61 (s, 1H).
IR(KBr) : 3100, 1627, 1466, 1319 $cm^{-1}$.

Méthode F : Préparation de l'acide 1-cyclopropyl-6,8-difluoro-1,4-dihydro-7-(3-méthyl-3-amino-1-azétidinyl)-4-oxo-3-quinoléincarboxylique

On chauffe à reflux pendant 2 heures un mélange de 0,23 g (0,82 mmoles) de l'acide 1-cyclopropyl-6,7,8-trifluoro-4-oxo-1,4-dihydro-3-quinoléincarboxylique, 0,26 g (1,64 mmoles) de dichlorhydrate de 3-méthyl-3-aminoazétidine et 0,5 ml de triéthylamine, dans 10 ml de pyridine. On filtre, on lave à l'eau et à l'éthanol et on obtient 0,250 g (87%) de l'acide 1-cyclopropyl-6,8-difluoro-1,4-dihydro-7-(3-méthyl-3-amino-1-azétidinyl)-4-oxo-3-quinoléincarboxylique de point de fusion et données spectroscopiques identiques à ceux du dérivé obtenu selon la méthode E.

EXEMPLE 17 :

Préparation de l'acide 1-cyclopropyl-6,8-difluoro-7-(3-acétoxy-1-azétidinyl)-1,4-dihydro-4-oxo-3-quinoléincarboxylique

On dissout 0,7 g (2 mmoles de l'acide 1-cyclopropyl-6,8-difluoro-7-(3-hydroxy-1-azétidinyl)-1,4- dihydro-4-oxo-3-quinoléincarboxylique (exemple 2) dans 20 ml de pyridine et on additionne lentement 0,64 9 (6,2 mmoles) d'anhydride acétique et on laisse à température ambiante pendant 24 heures. On dilue avec de l'eau et on filtre et lave le précipité. On obtient ainsi 0,54 g (68 %) de l'acide 1-cyclopropyl-6,8-difluoro-7-(3-acétoxy-1-azétidinyl)-1,4-dihydro-4-oxo-3-quinoléincarbo xylique de point de fusion 259-262 ° C.
Données spectroscopiques :
$^{1}$H RMN, δ, [DMSO-6d-TFA] : 1,2 (d, J=6Hz, 4H); 2,1 (s, 3H); 4,05 (q, J=6Hz, 1H); 4,4 (m, 2H); 4,8 (m, 2H); 5.3 (m, 1H), 7,7 (dd, J=13Hz, J'=2Hz, 1H); 8,60 (s, 1H). IR(KBr) : 1742, 1727, 1626, 1481 $cm^{-1}$.

EXEMPLE 18 :

Préparation de l'acide 1-cyclopropyl-6-fluoro-7-(3-hydroxy-1-azétidinyl)-1,4-dihydro-4-oxo-3-quinoléincarboxylique

On chauffe à 160 ° C un mélange de 1 g (3,5 mmoles) de l'acide 1-cyclopropyl-7-chloro-6-fluoro-4-oxo-1,4-dihydro-3-quinoléincarboxylique, 0,77 g (7,9 mmoles) de chlorhydrate de 3-hydroxyazétidine, 2,15 g (21,3 mmoles) de triéthylamine dans 10 ml de diméthylsulfoxyde, pendant 6 heures. On refroidit, on dilue avec de l'eau et on acidifie avec de l'acide acétique. On filtre, on recristallise de diméthylformamide et on obtient 0,3 g (27 %) de l'acide 1-cyclopropyl-6_fluoro-7-(3-hydroxy-1-azétidinyl)-1,4-dihydro-4-oxo-3-quinoléincarboxylique de point de fusion 296-8 ° C.
Données spectroscopiques :
$^{1}$H RMN, δ, [DMSO-6d, TFA]: 1,14 (m, 4H); 3,95 (m, 3H); 4,40 (m, 3H); 6,90 (d, 1H, J = 8 Hz); 7,7 (d, 1H, J = 12 Hz); 8,53 (s, 1H).
IR(KBr): 3406, 1703, 1632, 1524, 1340 $cm^{-1}$.

EXEMPLE 19 :

Préparation de l'acide 1-(4-fluorophényl)-6,8-difluoro-7-(3-méthyl-3-trifluoroacétamido-1-azétidinyl)-1,4-dihydro-4-oxo-3-quinoléincarboxylique

On chauffe à reflux pendant 3 heures un mélange de 1 g (3 mmoles) de l'acide 1-(4-fluorophényl)-6,7,8-trifluoro-1,4-dihydro-4)oxo-3-quinoléincarboxylique, 0,98 g (4,5 mmoles) d'hydrochlorure de 3-méthyl-3-trifluoroacétamidoazétidine et 0,6 g (6 mmoles de triéthylamine dans 10 ml de pyridine.
On évapore sous vide, on additionne de l'eau, on acidifie avec de l'acide acétique on filtre et on lave à l'eau. On obtient 1,25 g (84,5 %) de l'acide 1-(4-fluorophényl)-6,8-difluoro-7-(3-amino-3-trifluoroacétamido-1-azétidinyl) -1,4-dihydro-4-oxo-3-quinoléincarboxylique de point de fusion 198-203 ° C.
Données spectroscopiques :

$^1$H RMN, δ, [DMSO-6d, TFA]: 1,45 (s, 3H); 4,35 (m, 4H); 7,0-8,0 (m, 5H); 8,45 (s, 1H); 9,8 (s, 1H).
IR(KBr); 3400, 1734, 1701, 1627, 1489 $cm^{-1}$.

EXEMPLE 20 :

Préparation de l'acide 1-(4-fluorophényl)-6,8-difluoro-7-(3-amino-3-méthyl-1-azétidinyl)-1,4-dihydro-4-oxo-3-quinoléincarboxylique

On chauffe à reflux pendant 3 heures une solution de 1,25 g (2,5 mmoles) de l'acide 1-(4-fluorophényl)-6,8-difluoro-7-(3-amino-3-trifluoroacétamido-1-azétidinyl)-1,4 dihydro-4-oxo-3-quinoléincarboxylique (exemple 19), 8 ml de soude 1N et 10 ml d'eau. On refroidit, on filtre, on additionne de l'acide acétique, on filtre, on lave à l'eau et à l'éthanol froid. On obtient 0,8 g (72 %) de l'acide 1-(4-fluorophényl)-6,8-difluoro-7-(3-amino-3-méthyl-1-azétidinyl)-1,4-dihydro-4-oxo-3-quinoléincarboxylique de point de fusion 272-7 ° C.
Données spectroscopiques :
$^1$H RMN, δ, [DMSO-6d]: 1,40 (s, 3H); 4,1 (élargie, 4H); 7,4 (m, 2H); 7,7 (m, 3H); 8,36 (s, 1H).
IR(KBr) : 3400, 1728, 1626, 1466, 1325 $cm^{-1}$.

EXEMPLE 21 :

Préparation de l'acide 1-éthyl-6,8-difluoro-1,4-dihydro-4-oxo-7-(3-méthyl-3-trifluoroacétamido-1-azétidinyl)-3-quinoléincarboxylique

On chauffe à reflux pendant 3 heures une solution de 1,1g (4 mmoles) de l'acide 1-éthyl-6,7,8-trifluoro-1,4-dihydro-4-oxo-3-quinoléincarboxylique, 1,32 g (6 mmoles) de chlorhydrate de 3-méthyl-3-trifluoroacétamidoazétidine et 0,8 g (8 mmoles) de triéthylamine dans 10 ml de pyridine. On refroidit, on filtre et on lave à l'eau acidifiée avec un peu d'acide acétique. On obtient ainsi 0,65 g (37%) de l'acide 1-éthyl-6,8-difluoro-1,4-dihydro-4-oxo-7-(3-méthyl-3-trifluoroacétamido-1-azétidinyl)-3-quinoléincarboxylique de point de fusion 196-210 ° C.
Données spectroscopiques :
$^1$H RMN, δ, [DMSO-6d, TFA]: 1,45 (m, 3H); 1,60 (s, 3H); 4,51 (m, 6H); 7,68 (d, 1H, J = 13Hz); 8,76 (s, 1H); 9,80 (m, 1H).
IR(KBr) : 3400, 1724, 1707, 1629, 1497 $cm^{-1}$.

EXEMPLE 22 :

Préparation de l'acide 1-éthyl-6,8-difluoro-7-(3-amino-3-méthyl-1-azétidinyl)-1,4-dihydro-4-oxo-3-quinoléincarboxylique

On maintient à reflux pendant 3 heures un mélange de 0,65 g (1,5 mmoles) de l'acide 1-éthyl-6,8-difluoro-1,4-dihydro-4-oxo-7-(3-méthyl-3-trifluoroacétamido-1-azétidinyl)-3-quinoléincarboxylique (exemple 21), 2 ml de soude 10 % et 3 ml d'éthanol dans 10 ml d'eau. On filtre à chaud, on refroidit et on acidifie avec de l'acide acétique, on filtre et on lave à l'eau. On obtient 0,48 g (95 %) de l'acide 1-éthyl-6,8-difluoro-7-(3-amino-3-méthyl-1-azétidinyl)-1,4-dihydro-4-oxo-3-quinoléincarboxylique de point de fusion 293-6 ° C.
Données spectroscopiques :
$^1$H RMN, δ, [DMSO-6d, TFA]: 1,41 (m, 3H); 1,60 (s, 3H); 4,4 (m, 6H); 7,76 (d, 1H, J = 13Hz); 8,43 (m, 2H); 8,77 (s, 1H).
IR(KBr): 3400, 1723, 1628, 1467 $cm^{-1}$.

EXEMPLE 23 :

Préparation de l'acide 1-cyclopropyl-6,8-difluoro-7-(3-amino-1-azétidinyl)-1,4-dihydro-4-oxo-3-quinoléincarboxylique.

On chauffe un mélange de 1 g (3,2 mmoles) de 1-cyclopropyl-6,7,8-trifluoro-4-oxo-1,4-dihydro-3-quinoléincarboxylate d'éthyle, 0,98 g (4,8 mmoles) de chlorhydrate de 3-trifluoroacétamidoazétidine et 2 ml de triéthylamine dans 15 ml de diméthylsulfoxide, pendant 4 heures à 80-5 ° C. On dilue avec de l'eau et on extrait au chloroforme. On lave la phase organique avec de l'eau, on évapore et on obtient 0,31 g (22 %) de 1-cyclopropyl-6,8-difluoro-4-oxo-1,4-dihydro-7-3-trifluoroacétamido-1-azétidinyl)4-oxo-3-quinoléincarboxylate

d'éthyle qui est ensuite hydrolysé en chauffant un mélange de 0,1 g (0,22 mmoles) de cet ester avec 5 ml de soude 1N avec 5 ml d'éthanol. On refroidit, on évapore et on acidifie avec de l'acide acétique. On filtre, on lave à l'eau et on obtient 70 mg (96 %) de l'acide 1-cyclopropyl-6,8-difluoro-7-(3-amino-1-azétidinyl)-1,4-dihydro-4-oxo-3-quinoléincarboxylique de point de fusion 214-6° C.
Données spectroscopiques :
$^1$H RMN, δ, [DMSO-6d, TFA]: 1,0 (m, 4H); 3,40 (m, 1H); 3,9 (m, 1H); 4,45 (m, 4H); 7,5 (d, 1H, J = 7Hz); 8,3 (élargie, 2H); 8,5 (s, 1H).
IR(KBr): 3420, 2950, 1620, 1470, 1320 $cm^{-1}$.

EXEMPLE 24

Préparation de l'acide 1-cyclopropyl-6,8-difluoro-7-(3-trifluoroacétamidométhyl-3-méthyl-1-azétidinyl)-1,4-dihydro-4-oxo-3-quinoléincarboxylique

On chauffe à reflux pendant 3 heures un mélange de 1 g (3,5 mmoles) de l'acide 1-cyclopropyl-6,7,8-trifluoro-4-oxo-1,4-dihydro-3-quinoléincarboxylique, 1,32 g (5,7 mmoles) de chlorhydrate de 3-méthyl-3-trifluoroacétamidométhylazétidine et 2,3 g de triéthylamine dans 12 ml de pyridine. On évapore, on additionne de l'eau, on filtre, et on obtient 1,6 g (100 %) de l'acide 1-cyclopropyl-6,8-difluoro-7-(3-trifluoroacétamidométhyl-3-méthyl-1-azétidinyl)-1,4-dihydro-4-oxo-3-quinoléincarboxylique de point de fusion 232-7° C.
Données spectroscopiques :
$^1$H RMN, δ, [DMSO-6d,]: 1,25 (m, 7H); 3,5 (s, 2H); 4,20 (m, 5H); 7,62 (d, 1H, J = 13Hz); 8,56 (s, 1H); 9,27 (élargie, 1H)
IR(KBr): 3300, 1728, 1719, 1628, 1487, 1483 $cm^{-1}$

EXEMPLE 25

Préparation de l'acide 1-cyclopropyl-6,8-difluoro-7-(3-N'-éthyl-trifluoroacétamidométhyl-3-méthyl-1-azétidinyl)-1,4 dihydro-4-oxo-3-quinoléincarboxylique

On chauffe à reflux pendant 3 heures un mélange de 1,5 g (5,3 mmoles) de l'acide 1-cyclopropyl-6,7,8-trifluoro-1,4-dihydro-4-oxo-3-quinoléincarboxylique, 2,1 g (8 mmoles) de chlorhydrate de 3-méthyl-3-(N'-éthyl trifluoroacétamido méthyl)azétidine et 3,3 g de triéthylamine dans 15 ml de pyridine, on évapore, on additionne de l'eau, on filtre et on lave à l'eau et à l'éthanol. On obtient 1,8 g (70 %) de l'acide 1-cyclopropyl-6,8-difluoro-7-(3-N'-éthyl-trifluoroacétamidométhyl-3-méthyl-1-azétidinyl)-1,4-dihydro-4-oxo-3-quinoléincarboxylique, de point de fusion 210-2° C.
Données spectroscopiques :
$^1$H RMN, δ, [DMSO-6d,]: 1,25 (m, 10H); 3,48 (q, 2H, J = 7Hz); 3,72 (s, 2H); 4,18 (m, 5H); 7,67 (d, 1H, J = 13Hz); 8,58 (s, 1H).
IR(KBr): 1725, 1701, 1627, 1530, 1470 $cm^{-1}$.

EXEMPLE 26 :

Préparation de l'acide 1-cyclopropyl-6,8-difluoro-1,4-dihydro-4-oxo-7-(3-aminométhyl-3-méthyl-1-azétidinyl)-3-quinoléincarboxylique

On chauffe à reflux une solution de 1,5 g (3,3 mmoles) de l'acide 1-cyclopropyl-6,8-difluoro-7-(3-trifluoroacétamidométhyl-3-méthyl-1-azétidinyl)-1,4-dihydro-4-oxo-3-quinoléincarboxylique (exemple 24), 15 ml de soude 1N et 6 ml d'éthanol, pendant 3 heures, et on évapore sous vide. On additionne de l'acide acétique, on filtre, on lave à l'eau et on obtient 0,88 g (74 %) de l'acide 1-cyclopropyl-6,8-difluoro-1,4-dihydro-4-oxo-7-(3-aminométhyl-3-méthyl-1-azétidinyl)-3-quinoléincarboxylique de point de fusion 268-70° C.
Données spectroscopiques :
$^1$H RMN, δ, [DMSO-6d,]: 1.16 (d, 4H, J = 6,2Hz); 1,28 (s, 3H); 2,74 (s, 2H); 4,1 (m, 5H); 7,65 (d, 1H, J = 13Hz); 8,55 (s, 1H).
IR(KBr): 3400, 1725, 1627, 1465, 1455, 1322 $cm^{-1}$.

EXEMPLE 27 :

Préparation de l'acide 1-cyclopropyl-6,8-difluoro-7-(3-éthylaminométhyl-3-méthyl-1-azétidinyl)-1,4-dihydro-4-oxo-3-quinoléincarboxylique

On chauffe à reflux pendant 3 heures une solution de 1,7 g (3,5 mmoles) de l'acide 1-cyclopropyl-6,8-difluoro-7-(3-N'-éthyl-trifluoroacétamidométhyl-3-méthyl-1-azétidinyl)-1,4-dihydro-4-oxo-3-quinoléincarboxylique (exemple 25), 15 ml de soude 1N et 6 ml d'éthanol et on évapore sous vide. On refroidit, on additionne de l'acide acétique, on filtre, on lave à l'eau et on obtient 1,08 g (80 %) de l'acide 1-cyclopropyl-6,8-difluoro-7-(-3-éthylaminométhyl-3-méthyl 1-azétidinyl)-1,4-dihydro-4-oxo-3-quinoléincarboxylique, de point de fusion 250-5 ° C.
Données spectroscopiques
$^1$H RMN, $\delta$, [DMSO-$d_6$,]: 1,1 (m, 7H); 1,31 (s, 3H); 2,7 (m, 4H); 4,1 (m, 5H); 7,63 (d, 1H, J = 13Hz); 8,55 (s, 1H).
IR(KBr): 3440, 1615, 1475, 1400, 1320 $cm^{-1}$.

EXEMPLE 28 :

Préparation de l'acide 1-cyclopropyl-6-fluoro-7-(3-trifluoroacétamidométhyl-3-méthyl-1-azétidinyl)-1,4-dihydro-4-oxo-3-quinoléincarboxylique

On chauffe à reflux pendant 2 heures un mélange de 0,7 g (2,6 mmoles) de l'acide 1-cyclopropyl-6,7-difluoro-4-oxo-1,4-dihydro-3-quinoléincarboxylique, 0,92 g (3,96 mmoles) de chlorhydrate de 3-méthyl-3-trifluoroacétamidométhylazétidine et 1,6 g de triéthylamine dans 12 ml de pyridine. On évapore, on additionne de l'eau, on filtre, et on obtient 1,05 g (90 %) de l'acide 1-cyclopropyl-6-fluoro-7-(3-trifluoroacétamidométhyl-3-méthyl-1-azétidinyl)-1,4-dihydro-4-oxo-3-quinoléincarboxylique de point de fusion 265-72 ° C.
Données spectroscopiques :
$^1$H RMN, $\delta$, [DMSO-6d,]: 1,28 (m, 7H); 3,53 (s, 2H); 4,0 (m, 5H); 6.85(d, 1H, J = 6,9Hz); 7,76 (d, 1H, J = 12,9Hz); 8,56 (s, 1H)
IR(KBr): 3300, 1725, 1720, 1630, 1487, 1517, 1474 $cm^{-1}$

EXEMPLE 29 :

Préparation de l'acide 1-cyclopropyl-6-fluoro-7-(3-N'-éthyl-trifluoroacétamidométhyl-3-méthyl-1-azétidinyl)-1,4-dihydro-4-oxo-3-quinoléincarboxylique

On chauffe à reflux pendant 2 heures un mélange de 0,7 g (2,6 mmoles) de l'acide 1-cyclopropyl-6,7-difluoro-1,4-dihydro-4-oxo-3-quinoléincarboxylique, 1,04 g (3,9 mmoles) de chlorhydrate de 3-méthyl-3-(N'-éthyl-trifluoroacétamido méthyl)azétidine et 1,6 g de triéthylamine dans 12 ml de pyridine, on évapore, on additionne de l'eau, on filtre et on lave à l'eau. On obtient 0,78 g (63 %) de l'acide 1-cyclopropyl-6-fluoro-7-(3-N'-éthyl-trifluoroacétamidométhyl-3-méthyl-1-azétidinyl-1,4-dihydro-4-oxo-3-quinoléincarboxylique, de point de fusion 230-6 ° C.
Données spectroscopiques :
$^1$H RMN, $\delta$, [DMSO-6d,]: 1.25 (m, 10H); 3,48 (q, 2H, J = 6,5Hz); 3,72 (s, 2H); 4,04 (m, 5H); 7,90 (d, 1H, J = 8Hz); 7,76 (d, 1H, J = 12,8Hz); 8,56 (s, 1H).
IR(KBr): 1721, 1701, 1631, 1519, 1474, 1450 $cm^{-1}$.

EXEMPLE 30 :

Préparation de l'acide 1-cyclopropyl-6-fluoro-1,4-dihydro-4-oxo-7-(3-aminométhyl-3-méthyl-1-azétidinyl)-3-quinoléincarboxylique

On chauffe à reflux une solution de 1,05 g (2,38 mmoles) de l'acide 1-cyclopropyl-6-fluoro-7-(3-trifluoroacétamidométhyl-3-méthyl-1-azétidinyl)-1,4-dihydro-4-oxo-3-quinoléincarboxylique (exemple 30), 15 ml de soude 1N et 6 ml d'éthanol, pendant 3 heures, et on évapore sous vide. On additionne de l'acide acétique, on filtre, on lave à l'eau et on obtient 0,7 g (85 %) de l'acide 1-cyclopropyl-6-fluoro-1,4-dihydro-4-oxo-7-(3-aminométhyl-3-méthyl-1-azétidinyl)-3-qunoléincarboxylique de point de fusion 274-9 ° C.
Données spectroscopiques :
$^1$H RMN, $\delta$, [DMSO-6d,]: 1,24 (m, 7H); 2,76 (s, 2H); 3,90 (m, 5H); 6,84 (d, 1H, J = 7,6Hz); 7,75 (d, 1H, J = 12,9Hz); 8,55 (s, 1H).

IR(KBr): 3400, 1721, 1631, 1520, 1470, 1395 $cm^{-1}$.

EXEMPLE 31 :

Préparation de l'acide 1-cyclopropyl-6-fluoro-7-(3-éthylaminométhyl-3-méthyl-1-azétidinyl)-1,4-dihydro-4-oxo-3-quinoléincarboxylique

On chauffe à reflux pendant 3 heures une solution de 0,78 g (3,5 mmoles) de l'acide 1-cyclopropyl -6-fluoro-7-(3-N'-éthyl-trifluoroacétamidométhyl-3-méthyl-1-azétidinyl) - 1,4-dihydro-4-oxo-3-quinoléin-carboxylique (exemple 31), 15 ml de soude 1N et 6 ml d'éthanol et on évapore sous vide. On refroidit, on additionne de l'acide acétique, on filtre, on lave à l'eau et on obtient 0,4 g (65%) de l'acide 1-cyclopropyl-6-fluoro-7-(-3éthylaminométhyl-3-méthyl-1-azétidinyl)-1,4-dihydro-4-oxo-3quinoléincarboxylique, de point de fusion 221-6 ° C.

Données spectroscopiques :
$^1$H RMN, δ, [DMSO-$d_6$,]: 1.15 (m, 10H); 2,68 (m, 4H); 3,9 (m, 5H); 6.84 (d, 1H, J = 7,6Hz); 7,75 (d, 1H, J = 12,8Hz); 8,55 (s, 1H).
IR(KBr): 3420, 1629, 1619, 1578, 1517, 1484, 1402 $cm^{-1}$.

EXEMPLE 32 :

Préparation de l'acide 1-(2,4-difluorophényl)-6,8-difluoro-7-(3-méthyl-3-trifluoroacétamido-1-azétidinyl)-1,4-dihydro-4-oxo-3-quinoléincarboxylique

On chauffe à reflux pendant 2 heures un mélange de 0,8 g (2,3 mmoles) de l'acide 1-(2,4-difluorophényl)-6,7, 8-trifluoro-1,4-dihydro-4-oxo-3-quinoléincarboxylique, 0,8 g (3,7 mmoles) d'hydrochlorure de 3-méthyl-3-trifluoroacétamidoazétidine et 0,6 g (6 mmoles) de triéthylamine dans 15 ml de pyridine.

On évapore sous vide, on additionne de l'eau, on acidifie avec de l'acide acétique on filtre et on lave à l'eau. On obtient 1,10 g (57 %) de l'acide 1-(2,4-difluorophényl)-6,8-difluoro-7-(3-amino-3-trifluoro acétamido-1-azétidinyl)-1,4-dihydro-4-oxo-3-quinoléincarboxylique de point de fusion 190-6 ° C.

Données spectroscopiques :
$^1$H RMN, δ, [DMSO-6d, TFA]: 1,54 (s, 3H); 4.4 (m, 4H); 7,0-8,0 (m, 4H); 8,60 (s, 1H); 9.7 (s, 1H).
IR(KBr); 3400, 1720, 1711, 1626, 1459 $cm^{-1}$.

EXEMPLE 33 :

Préparation de l'acide 1-(2,4-difluorophényl)-6,8-difluoro-7-(3-amino-3-méthyl-1-azétidinyl)-1,4-dihydro-4-oxo-3-quinoléincarboxylique

On chauffe à reflux pendant 3 heures une solution de 1,1 g (2,1 mmoles) de l'acide 1-(2,4-difluorophényl)-6,8-difluoro-7-(3-méthyl-3-trifluoroacétamido-1-azétidinyl)-1,4-dihydro-4-oxo-3-quinoléincarboxylique (exemple 34), 4 ml de soude 10 %, 5 ml d'éthanol et 25 ml d'eau. On refroidit, on filtre, on additionne de l'acide acétique, on filtre, on lave à l'eau et à l'éthanol froid. On obtient 0,2 g (22 %) de l'acide 1-(2,4-difluorophényl)-6,8-difluoro-7-(3-amino-3-méthyl-1-azétidinyl)-1,4-dihydro-4-oxo-3-quinoléincarboxylique de point de fusion 185-6 ° C.

Données spectroscopiques :
$^1$H RMN, δ, [DMSO-6d, TFA]: 1,54 (s, 3H); 4,31 (élargie, 4H); 7,3 - 8.1 (m, 4H); 8,48 (élargie, 2H); 8,62 (s, 1H).
IR(KBr): 3410, 1729, 1625, 1510, 1461 $cm^{-1}$.

EXEMPLE 34 :

Préparation de l'acide 1-éthyl-6-fluoro-7-(3-amino-3-méthyl-1-azétidinyl)-1,4-dihydro-4-oxo-3-quinoléincarboxylique.

On chauffe à reflux pendant 2 heures un mélange de 0,8 g (3,16 mmoles) de l'acide 1-éthyl-6,7-difluoro-4-oxo-1,4-dihydro-3-quinoléincarboxylique, 1,5 g (6,8mmoles) de chlorhydrate de 3-méthyl-3-trifluoroacétamidoazétidine et 1 g (10 mmoles) de triéthylamine dans 15 ml de pyridine.

On dilue avec de l'eau et on extrait au chloroforme. On lave la phase organique avec de l'eau, on

évapore et on obtient 1,0 g (76%) de l'acide 1-éthyl-6-fluoro-4-oxo-1,4-dihydro-7-(3-méthyl-3-trifluoroacétamido-1-azétidinyl)-4-oxo-3-quinoléincarboxylique qui est ensuite hydrolysé en chauffant un mélange de 1,0 g (2,4 mmoles) de cet acide avec 3 ml de soude 10 % et 20 ml d'eau à reflux pendant 3 heures. On refroidit, et on acidifie avec de l'acide acétique. On filtre, on lave à l'eau, et on obtient 370 mg (48 %) de l'acide 1-éthyl-6-fluoro-7-(3-méthyl-3-amino-1-azétidinyl)-1,4-dihydro-4-oxo-3-quinoléincarboxylique de point de fusion 280-3°C.
Données spectroscopiques :
$^1$H RMN, δ, [DMSO-6d]: 1,46 (m, 6H); 4,00 (m, 4H); 4,50 (m, 2H); 6.6 (d, 1H, J = 7,8Hz); 7,82 (d, 1H, J = 12,9Hz); 8,87 (s, 1H).
IR(KBr): 3420, 1709, 1631, 1430, 1360 $cm^{-1}$.

EXEMPLE 35 :

Préparation de l'acide 1-(2-fluoroéthyl)-6-fluoro-7-(3-méthyl-3-amino-1-azétidinyl)-1,4-dihydro-4-oxo-3-quinoléincarboxylique

On chauffe à reflux pendant 2 heures un mélange de 0,8 g (2,5 mmoles) de l'acide 1-(2-fluoroéthyl)-6,7-difluoro-1,4-dihydro-4-oxo-3-quinoléincarboxylique, 1,37 g (6,3 mmoles) d'hydrochlorure de 3-méthyl-3-trifluoroacétamidoazétidine et 1,1 g (10 mmoles) de triéthylamine dans 10 ml de pyridine.

On évapore sous vide on extrait au chlorure de méthylène, on lave à l'eau et on obtient 1,2 g (99 %) de l'acide 1-(2-fluoroéthyl)-6-fluoro-7-(3-méthyl-3-trifluoroacétamido-1-azétidinyl)-1,4-dihydro-4-oxo-3-quinoléincarboxylique de point de fusion 225-8°C.

On hydrolyse ce produit en chauffant à reflux pendant 2 heures une solution de 1,1 g (2,3 mmoles) de cette trifluoroacétamide dans 25 ml d'eau additionnés de 3 ml de soude 10%. On filtre à chaud,on acidifie avec de l'acide acétique, on filtre, on lave à l'eau et à l'éthanol et on obtient 0,3 g (34 %) de l'acide 1-(2-fluoroéthyl)-6-fluoro-7-(3-méthyl-3-amino-1-azétidinyl)-1,4-dihydro-4-oxo-3-quinoléincarboxylique de point de fusion 265-70°C.
Données spectroscopiques :
$^1$H RMN, δ, [DMSO-6d]: 1,47 (s, 3H); 4,04 (m, 4H); 4,65 (m, 2H); 5,02 (s, 2H); 6.60 (d, 1H, J = 7,3Hz); 7,81 (d, 1H, J = 12,9Hz) 8.78 (s, 1H)
IR(KBr): 3480, 1719, 1632, 1463 $cm^{-1}$.

EXEMPLE 36 :

Préparation de l'acide 1-(2,4-difluorophényl)-6-fluoro-7-(3-méthyl-3-amino-1-azétidinyl)-1,4-dihydro-4-oxo-3-quinoléincarboxylique

On chauffe à reflux pendant 2 heures un mélange de 1,3 g (6 mmoles) de chlorhydrate de 3-méthyl-3-trifluoroacétamidoazétidine, 0,8 g (2,4 mmoles) de l'acide 1-(2,4-difluorophényl)-6,7-difluoro-1,4-dihydro-4-oxo-3-quinoléincarboxylique, et 0,8 g (8 mmoles) de triéthylamine dans 20 ml de pyridine. On évapore, on extrait au chlorure de méthylène et on obtient 1,1 g (92 %) de l'acide 1-(2,4-difluorophényl)-6-fluoro-7-(3-méthyl-3-trifluoroacétamido-1-azétidinyl)-1,4-dihydro-4-oxo-3-quinoléincarboxylique qui est ensuite hydrolisé en l'ajoutant à une solution de 3 ml de soude 10 % dans 20 ml d'eau et qui est portée à reflux pendant 2 heures. On filtre à chaud, on acidifie avec de l'acide acétique, on filtre et on lave à l'eau et à l'éthanol. On obtient 0,27 g (30 %) de l'acide 1-(2,4-difluorophényl)-6-fluoro-7-(3-méthyl-3-amino-1-azétidinyl)-1,4-dihydro-4-oxo-3-quinoléincarboxylique de point de fusion 210-6°C.
Données spectroscopiques :
$^1$H RMN, δ, [DMSO-6d, TFA]; 1,56 (s, 3H); 4,00 (m, 4H); 5,72 (d, 1H, J = 7,1Hz); 7,3 - 8,1 (m, 4H); 8,44 (élargie, 2H) 8.70 (s, 1H).
IR(KBr): 3400, 1725, 1630, 1509, 1474 $cm^{-1}$.

EXEMPLE 37 :

Préparation de l'acide 1-cyclopropyl-6,8-difluoro-1,4-dihydro-4-oxo-7-(3-méthyl -3-diméthylamino-1-azétidinyl) -3-quinoléincarboxylique.

On chauffe à reflux pendant 3 heures une solution de 1,5 g (5,3 mmoles) d'acide 1-cyclopropyl-6,7,8-trifluoro-1,4-dihydro-4-oxo-3-quinoléincarboxylique, 1,5 g (8 mmoles) de chlorhydrate de 3-méthyl-3-dimé-

thylaminoazétidine et 3,3 g (33 mmoles) de triéthylamine dans 15 ml de pyridine. On refroidit, on évapore sous vide, on additionne de l'eau, on basifie avec de la soude 10 %, on filtre, on acidifie avec de l'acide acétique et on obtient un précipité qu'on basifie légèrement avec de l'ammoniaque. On chauffe pour évaporer l'excès d'ammoniaque et on obtient 1,85 g (92 %) de l'acide 1-cyclopropyl-6,8-difluoro-1,4-dihydro-4-oxo-7-(3-méthyl-3-diméthylamino-1-azétidinyl)-3-quinoléincarboxylique de point de fusion 280-4° C.
Données spectroscopiques :
$^1$H RMN, δ, [DMSO-6d, TFA]: 1,19 (d, 4H, J = 6,5Hz); 1,71 (s, 3H); 2,82 (s, 6H); 4,03 (m, 1H); 4,52 (m, 4H); 7,76 (dd, 1H, J = 12,8Hz, J' = 1,8Hz); 8,62 (s, 1H)
IR(KBr): 1723, 1626, 1552, 1492, 1451 $cm^{-1}$.

EXEMPLE 38 :

Préparation de l'acide 1-cyclopropyl-6-fluoro-7-(3-amino-3-méthyl-1-azétidinyl)-1,4-dihydro-4-oxo-3-quinoléincarboxylique.

On chauffe à reflux pendant 2 heures un mélange de 0,8 g (3,0 mmoles) de l'acide 1-cyclopropyl-6,7-difluoro-4-oxo-1,4-dihydro-3-quinoléincarboxylique, 1,7 g (7,8 mmoles) de chlorhydrate de 3-méthyl-3-trifluoroacétamidoazétidine et 1,4 g de triéthylamine dans 15 ml de pyridine. On évapore, on additionne de l'eau, on filtre, et on obtient 0,55 g (42 %) de l'acide 1-cyclopropyl-6-fluoro-7-(3-trifluoroacétamido-3-méthyl-1-azétidinyl)-1,4-dihydro-4-oxo-3-quinoléincarboxylique qui est ensuite hydrolysé en le chauffant avec une solution de 3 ml de soude 10 % dans 10 ml d'eau, à reflux, pendant 2 heures. On porte le volume à moitiée, on additionne quelques gouttes d'acide acétique, on filtre et on lave à l'eau. On obtient ainsi 0,36 g (84 %) de l'acide 1-cyclopropyl-6-fluoro-7-(3-amino-3-méthyl-1-azétidinyl)-1,4-dihydro-4-oxo-3-quinoléincarboxylique de point de fusion 293-5° C.
Données spectroscopiques :
$^1$H RMN, δ, [DMSO-6d]; 1,22 (m, 4H); 1,45 (s, 3H); 3,69 (m, 1H); 4,0 (m, 4H); 6,85 (d, 1H, J = 7,8 Hz) 7,75 (d, 1H, J = 12,9 Hz); 8,55 (s, 1H).
IR(KBr): 3340, 1722, 1630, 1528, 1471 $cm^{-1}$.

EXEMPLE 39 :

Préparation de l'acide 1-(2-fluoroéthyl)-6,8-difluoro-7-(3-amino-3-méthyl-1-azétidinyl)-1,4-dihydro-4-oxo-3-quinoléincarboxylique

On chauffe à reflux pendant 2 heures un mélange de 0,8 g (2,8 mmoles) de l'acide 1-(2-fluoroéthyl )-6,7,8-trifluoro-4-oxo-1,4-dihydro-3-quinoléincarboxylique, 1,3 g (6 mmoles) de chlorhydrate de 3-méthyl-3-trifluoroacétamidoazétidine et 1 g (10 mmoles) de triéthylamine dans 15 ml de pyridine.

On évapore sous vide, on extrait au chlorure de méthylène, on filtre, on évapore et on obtient 1,2 g (95 %) de l'acide 1-(2-fluoroéthyl)-6,8-difluoro-4-oxo-1,4-dihydro-7-(3-méthyl-3-trifluoroacétamido-1-azétidinyl)-4-oxo-3-quinoléincarboxylique de point de fusion 205-15° C qui est ensuite hydrolysé en chauffant un mélange de 1,0 g (2,0 mmoles) de cet acide avec 3 ml de soude 10 % et 20 ml d'eau à reflux pendant 3 heures. On refroidit, et on acidifie avec de l'acide acétique. On filtre, on lave à l'eau, et on obtient 380 mg (48 %) de l'acide 1-(2-fluoroéthyl)-6,8-difluoro-7-(3-méthyl-3-amino-1-azétidinyl)-1,4-dihydro-4-oxo-3-quinoléincarboxylique de point de fusion 281-4° C.
Données spectroscopiques :
$^1$H RMN, δ, [DMSO-6d]: 1,41 (s, 3H); 4,17 (m, 4H); 4,62 (m, 2H); 5,04 (m, 2H); 7,66 (d, 1H, J = 12,3Hz); 8,73 (s, 1H).
IR(KBr): 3410, 1725, 1629, 1614, 1474 $cm^{-1}$.

EXEMPLE 40 :

Préparation de l'acide 1-(4-fluorophényl)-6-fluoro-7-(3-amino-3-méthyl-1-azétidinyl)-1,4-dihydro-4-oxo-3-quinoléincarboxylique

On chauffe à reflux pendant 3 heures un mélange de 0,8 g (2,5 mmoles) de l'acide 1-(4-fluorophényl)-6,7-difluoro-1,4-dihydro-4-oxo-3-quinoléincarboxylique, 1,3 g (6 mmoles) de chlorhydrate de 3-méthyl-3-trifluoroacétamidoazétidine, et 1 g de triéthylamine, dans 20 ml de pyridine. On évapore, on extrait au

chlorure de méthylène, on filtre et évapore. On obtient 1,1 g de 1-(4-fluorophényl)-6-fluoro-7-(3-trifluoroacétamido-3-méthyl-1-azétidinyl)-1,4-dihydro-4-oxo-3-quinoléincarboxylique de point de fusion 146-151 ° C, qui est ensuite hydrolysé de façon semblable à celle de l'exemple 41 et on obtient 0,5 g (56 %) de l'acide 1-(4-fluorophényl)-6-fluoro-7-(3-amino-3-méthyl-1-azétidinyl)-1,4-dihydro-4-oxo-3-quinoléincarboxylique de point de fusion 270-6 ° C.
Données spectroscopiques :
$^1$H RMN, δ, [DMSO-6d]; 1,35 (s, 3H); 2,9 (m, 2H); 3,76 (m, 4H); 5,70 (d, 1H, J = 7,9Hz); 7,2 - 7,9 (m, 5H); 8,48 (s, 1H).
IR(KBr): 3420, 1720, 1630, 1505 $cm^{-1}$.

EXEMPLE 41 :

Préparation de l'acide 1-cyclopropyl-6,8-difluoro-7-(3-dimétylamino-1-azétidinyl)-1,4-dihydro-4-oxo-3-quinoléincarboxylique

On chauffe à reflux pendant 3 heures un mélange de 1,5 g (5,3 mmoles) de l'acide 1-cyclopropyl-6,7,8-trifluoro-1,4-dihydro-4-oxo-3-quinoléincarboxylique, 1,4 g (8 mmoles) de dichlorhydrate de 3-diméthylaminoazétidine et 6,6 g de triéthylamine dans 15 ml de pyridine. On évapore, on additionne de l'eau, on basifie avec de la soude 1N on chauffe, on filtre à chaud, on acidifie avec de l'acide acétique, on filtre et on lave à l'eau. On obtient 1,7 g (88 %) de l'acide 1-cyclopropyl-6,8-difluoro-7-(3-diméthylamino-1-azétidinyl)-1,4-dihydro-4-oxo-3-quinoléincarboxylique de point de fusion 256-60 ° C.
Données spectroscopiques :
$^1$H RMN, δ, [DMSO-6d]; 1,18 (d, 4H, J = 5,7Hz); 2,16 (s, 6H); 3,28 (m, 1H); 4,24 (m, 5H); 7,68 (d, 1H, J = 12,9Hz); 8,57 (s, 1H).
IR(KBr): 1718, 1629, 1528, 1459, 1439 $cm^{-1}$.

EXEMPLE 42 :

Préparation de l'acide 1-cyclopropyl-6-fluoro-7-(3-diméthylamino-1-azétidinyl)-1,4-dihydro-4-oxo-3-quinoléincarboxylique.

On chauffe à reflux pendant 2 heures un mélange de 0,25 g (1,32 mmoles) de l'acide 1-cyclopropyl-6,7-difluoro-1,4-dihydro-4-oxo-3-quinoléincarboxylique, 0,34 g (2 mmoles) de dichlorhydrate de 3-diméthylaminoazétidine et 3,3 g (3,3 mmoles) de triéthylamine, dans 10 ml de pyridine. On évapore sous vide, on additionne de l'eau, on basifie avec de la soude 1N, on chauffe, on filtre à chaud, on acidifie avec de l'acide acétique, on filtre, on lave à l'eau et on obtient 0,4 g (88 %) de l'acide 1-cyclopropyl-6-fluoro-7-(3-diméthylamino-1-azétidinyl)-1,4-dihydro-4-oxo-3-quinoléincarboxylique de point de fusion 255-61 ° C.
Données spectroscopiques :
$^1$H RMN, δ, [DMSO-6d]; 1,21 (m, 4H); 2,18 (s, 6H); 3,34 (m, 1H); 3.70 (m, 1H); 4,14 (m, 4H); 6,88 (d, 1H, J = 7,5Hz); 7,76 (d, 1H, J = 12,9Hz); 8,56 (s, 1H).
IR(KBr): 1709, 1631, 1522, 1468, 1459 $cm^{-1}$.

EXEMPLE 43 :

Préparation du chlorhydrate de l'acide 1-cyclopropyl-6,8-difluoro-7-(3-amino-3-méthyl-1-azétidinyl)-1,4-dihydro-4-oxo-3-quinoléincarboxylique

On suspend 0,5 g (1,4 mmoles) de l'acide 1-cyclopropyl-6,8-difluoro-7-(3-amino-3-méthyl-1-azétidinyl)-1,4-dihydro-4-oxo-3-quinoléincarboxylique dans 10 ml de méthanol et on additionne un excès de solution méthanolique d'acide chlorhydrique gazeux. On agite pendant 30 minutes, on additionne de l'éther éthylique et de l'éther de pétrole. On filtre, on lave à l'éther éthylique, on sèche à chaud et on obtient 0,45 g (82 %) de chlorhydrate de l'acide 1-cyclopropyl-6,8-difluoro-7-(3-amino-3-méthyl-1-azétidinyl)-1,4-dihydro-4-oxo-3-quinoléincarboxylique de point de fusion 249-250 ° C.
Données spectroscopiques :
$^1$H RMN, δ, [DMSO-6d]; 1,20 (m, 4H); 1,65 (m, 3H); 3,29 (élargie $H_2O$) 4,05 (m, 1H); 4,46 (m, 4H); 7,76 (d, 1H, J = 12,8 Hz); 8,61 (s, 1H) 8,72 (élargie, 2H).
IR(KBr): 3431, 1719, 1629, 1531, 1462, 1333 $cm^{-1}$.

EXEMPLE 44 :

Préparation du sel de sodium de l'acide 1-cyclopropyl-6,8-difluoro-7-(3-amino-3-méthyl-1-azétidinyl)-1,4-dihydro-4-oxo-3-quinoléincarboxylique

On ajoute 95 mg (0,27 mmoles) de l'acide 1-cyclopropyl-6,8-difluoro-7-(3-amino-3-méthyl-1-azétidinyl)-1,4-dihydro-4-oxo-3-quinoléincarboxylique à une solution de 22,8 mg (0,27 mmoles) de bicarbonate de sodium dans 5 ml d'eau, on agite vigoureusement, on additionne quelques gouttes d'éthanol et on chauffe à 80 ° C pendant 18 heures. On évapore, on additionne de l'éthanol, on filtre et on lave à l'éthanol. On obtient 62 mg (63 %) du sel de sodium de l'acide 1-cyclopropyl-6,8-difluoro-7-(3-amino-3-méthyl-1-azétidinyl)-1,4-dihydro-4-oxo-3-quinoléincarboxylique de point de fusion > 300 ° C.
Données spectroscopiques :
$^1$H RMN, $\delta$, [$D_2O$]; 0,89 (m, 2H); 0,98 (m, 2H); 1,29 (s, 3H); 3,72 (m, 1H); 3,94 (m, 2H); 4,04 (m, 2H); 7,44 (dd, 1H, J = 12,93 Hz, J' = 1,45 Hz); 8,23 (s, 1H).
IR(KBr): 3400, 1620, 1462, 1400, $cm^{-1}$.

EXEMPLE 45 :

Préparation de l'acide 1-cyclopropyl-6,8-difluoro-7-3-méthylamino-1-azétidinyl)-1,4-dihydro-4-oxo-3-quinoléincarboxylique.

On chauffe à reflux pendant 2 heures un mélange de 0,85 g (3,0 mmoles) de l'acide 1-cyclopropyl-6,7,8-trifluoro-4-oxo-1,4-dihydro-3-quinoléincarboxylique, 1,3 g (6 mmoles) de chlorhydrate de 3-méthyl-3-trifluoroacétamidoazétidine et 0,8 g (8 mmoles) de triéthylamine dans 15 ml de pyridine.

On évapore sous vide, on acidifie avec de l'acide acétique aqueux et on obtient 1,1 g de l'acide 1-cyclopropyl-6,8-difluoro-4-oxo-1,4-dihydro-7-(3-méthyl-3-trifluoroacétamido-1-azétidinyl)-4-oxo-3-quinoléincarboxylique qui est ensuite hydrolisé en chauffant un mélange de 1,0 g (2,0 mmoles) de cet acide avec 3 ml de soude 10 % et 20 ml d'eau à reflux pendant 3 heures. On refroidit, et on acidifie avec de l'acide acétique. On filtre, on lave à l'eau, et on obtient 0,6 g (48 %) de l'acide 1-cyclopropyl-6,8-difluoro-7-(3-méthylamino-1-azétidinyl)-1,4-dihydro-4-oxo-3-quinoléincarboxylique de point de fusion 270-2 ° C.
Données spectroscopiques :
$^1$H RMN, $\delta$, [DMSO-6d]: 1,17 (d, 4H, J - 6,5Hz); 2,31 (m, 3H); 3,66 (m, 1H); 4,12 (m, 3H); 4,52 (m, 2H); 7,66 (dd, 1H, J = 12,3Hz, J' = 1,7Hz); 8,58 (s, 1H).
IR(KBr): 3468, 3387, 2912, 1718, 1629, 1617, 1472 $cm^{-1}$.

EXEMPLE 46 :

Préparation de l'acide 1-cyclopropyl-6-fluoro-7-(3-méthylamino-1-azétidinyl)-1,4-dihydro-4-oxo-3-quinoléincarboxylique

On chauffe à reflux pendant 3 heures un mélange de 0,5 g (2,0 mmoles) de l'acide 1-cyclopropyl-6,7-difluoro-1,4-dihydro-4-oxo-3-quinoléincarboxylique, 1,2 g (6 mmoles) de chlorhydrate de 3-méthyl-3-trifluoroacétamidoazétidine, et 0,8 g de triéthylamine, dans 20 ml de pyridine. On évapore, on additionne de l'eau glacée et on obtient 0,7 g de 1-cyclopropyl-6-fluoro-7-(3-méthyl-3-trifluorométhylacétamido-1-azétidinyl)-1,4-dihydro-4-oxo-3-quinoléincarboxylique qui est ensuite hydrolysé de façon semblable à celle de l'exemple 47 et on obtient 250 mg de l'acide 1-cyclopropyl-6-fluoro-7-(3-méthylamino-1-azétidinyl)-1,4-dihydro-4-oxo-3-quinoléincarboxylique de point de fusion 245-9 ° C. Données spectroscopiques :

EXEMPLE 47 :

Préparation de l'acide 1-cyclopropyl-6-fluoro-7-(3-amino-1-azétidinyl)-1,4-dihydro-4-oxo-3-quinoléincarboxylique

On chauffe à reflux pendant 2 heures un mélange de 1,2 g (4,53 mmoles) de l'acide 1-cyclopropyl-6,7-difluoro-1,4-dihydro-4-oxo-3-quinoléincarboxylique, 1,3 g (9,05 mmoles) de dichlorhydrate de 3-aminoazétidine et 0,5 ml de triéthylamine dans 15 ml de pyridine. On filtre, on lave à l'eau et à l'éthanol et on obtient 0,83 g (58 %) de l'acide 1-cyclopropyl-6-fluoro-7-(3-amino-1-azétidinyl)-1,4-dihydro-4-oxo-3-quinoléincarboxylique de point de fusion 246-7 ° C.

Données spectroscopiques :
$^1H$ RMN, δ, [DMSO-6d]; 1,28 (m, 4H); 3,87 (m, 4H); 4,40 (m, 2H); 6,60 (elargie, 2H); 6,86 (d, 1H, J = 8Hz); 7,77 (d, 1H, J = 13Hz); 8,56 (s, 1H).

L'activité pharmacologique antimicrobienne de ces composés a été étudiée selon les revendications exposées ci-dessous. Activité pharmacologique antimicrobienne (G.L. Daquet et Y.A. Chabbect, Techniques en bactériologie, Vol. 3, Flammarion Médecine-Sciences, Paris, 1972 et W.B. Hugo et A.D. Rusell, Pharmaceutical Microbiology, Blackwell Scientific Publications, London, 1977).

-Milieu de culture et solvant :

Agar d'antibiotiques n° 1 (Oxoid CM 327)
Bouillon de triptone-soja (Oxoid CM 129)
Solution physiologique Ringer 1/4 (Oxoid BR 52)
Agar Dextrose (BBL-11165)
NaOH 0,1 N

- Microorganismes :

"Bacillus subtilis" ATCC 6633
"Citrobacter freundii" ATCC 11606
"Enterobacter aerogenes" ATCC 15038
"Enterobacter cloacae" ATCC 23355
"Bacillus cereus" ATCC 1178
"Escherichia coli" ATCC 10799
"Escherichia coli" ATCC 23559
"Klebsiella pneumoniae" ATCC 10031
"Proteus Vulgaris " ATCC 8427
"Morg. morganii" ATC 8019
"Pseudomonas aeruginosa" ATCC 9721
"Pseudomonas aeruginosa" ATCC 10145
"Salmonella tiphymurium" ATCC 14028
"Salmonella tiphymurium" ATCC 6539
"Serratia marcescens" ATCC 13880
"Shigella flexnerii" ATCC 12022
"Staphylococcus epidermis ATCC 155-1
"Staphylococcus aureus" ATCC 25178
"Streptococcus faecalis"ATCC 10541

- Préparation des inoculations

Chacun des microorganismes est ensemencé par strie dans des tubes d'Agar d'antibiotiques n° 1, et mis en incubation pendant 20 heures à 37° C. Ensuite on prend une anse de culture, on ensemence dans un bouillon de Triptone-soja et on incube pendant 20 heures à 37° C. On dilue à 1/4 la culture obtenue avec une solution physiologique Ringer, de façon à obtenir une suspension normalisée de $10^7$-$10^9$ ufc/ml pour chaque organisme.

-Préparation du milieu contenant les dérivés de formule générale I

A partir d'une solution de 1000 µg/ml dans NaOH 0,1 N, chaque produit est dilué dans l'Agar Dextrose (préalablement fondu et maintenu à 50° C) par dilutions successives de façon à obtenir les concentrations suivantes : 64 - 32 - 16 - 8 - 4 - 2 - 1 - 0,5 - 0,25 - 0,125 µg de dérivé/ml du milieu.

Postérieurement, chaque concentration de chaque produit est répartie dans des boîtes de Petri de 10 cm de diamètre, à raison de 10 ml de milieu par boîte et autant de boîtes que de microorganismes à tester.

Une fois le milieu refroidi, les boîtes sont ensemencées avec les inoculations à raison de 0,4 ml d'inoculation par boîte. On les étend avec une anse de Driglasky et on recueille le surnageant. Les boîtes ensemencées sont incubées à 37° C pendant 20 heures.

Résultats

Les résultats obtenus sont décrits dans les tableaux suivants. L'activité des composés "in vitro" y est comparée à celle de l'acide pipémidique. Les concentrations sont données en µg/ml.

| MICROORGANISMES | EXEMPLES | | | | | |
|---|---|---|---|---|---|---|
| | Acide pipémidique | 2 | 5 | 6 | 7 | 8 |
| Bacillus subtilis ATCC 6633 | 8 | ≤0,015 | 8 | 0,06 | 0,06 | ≤0,03 |
| Bacillus cereus ATCC 11778 | 16 | 0,3 | 64 | 0,25 | 0,50 | 0,25 |
| Strep. faecalis ATCC 10541 | >64 | 0,5 | 64 | 0,50 | 4 | 1 |
| Staph. aureus ATCC 25178 | 64 | 0,06 | 64 | 0,25 | 1 | 0,25 |
| Staph. epidermidis ATCC 155-1 | 64 | 0,12 | 64 | 0,50 | 0,50 | 0,25 |
| Ps. aeruginosa ATCC 9721 | 32 | 1,0 | 64 | 2 | 4 | 1 |
| Ps. aeruginosa ATCC 10145 | 32 | 2,0 | 64 | 4 | 8 | 4 |
| Citr. freundii ATCC 11606 | 4 | 0,06 | 64 | 0,5 | 1 | 0,5 |
| Morg. morganii ATCC 8019 | 8 | 0,6 | 64 | 0,25 | 1 | 0,5 |
| Proteus vulgaris ATCC 8427 | 16 | 0,6 | 64 | 0,25 | 1 | 0,5 |
| Kleb. pneumoniae ATCC 10031 | 2 | ≤0,015 | 64 | 0,06 | 0,50 | 0,5 |
| Sal. typhimurium ATCC 14028 | 8 | 0,12 | 64 | 0,5 | 1 | 0,5 |
| Sal. typhi ATCC 6539 | 4 | 0,06 | 64 | 0,25 | 1 | 0,5 |
| Escherichia coli ATCC 10799 | 16 | 0,12 | 64 | 0,50 | 1 | 0,5 |
| Escherichia coli ATCC 23559 | 2 | 0,06 | 64 | 0,25 | 1 | 0,5 |
| Ent. aerogenes ATCC 15038 | 32 | 0,12 | 64 | 0,50 | 1 | 0,5 |
| Ent. cloacae ATCC 23355 | 8 | 0,06 | 64 | 0,25 | 1 | 0,5 |
| Serr. marcescens ATCC 13880 | 16 | 0,25 | 64 | 1 | 1 | 0,5 |
| Shigella flexnerii ATCC 12022 | 4 | 0,06 | 64 | 0,25 | 0,50 | 0,25 |

| MICROORGANISMES | EXEMPLES | | | | | |
|---|---|---|---|---|---|---|
| | 9 | 10 | 11 | 12 | 13 | 14 |
| Bacillus subtilis ATCC 6633 | 0,06 | 0,06 | ≤0,03 | 0,12 | 0,5 | ≤0,03 |
| Bacillus cereus ATCC 11778 | 1 | 0,12 | 0,12 | 0,5 | 0,5 | 0,25 |
| Strep. faecalis ATCC 10541 | 1 | 0,12 | 0,5 | 2 | 2 | 1 |
| Staph. aureus ATCC 25178 | 0,25 | 0,12 | 0,06 | 0,5 | 1 | 0,25 |
| Staph. epidermidis ATCC 155-1 | 1 | 0,12 | 0,06 | 0,5 | 0,5 | 0,12 |
| Ps. aeruginosa ATCC 9721 | 1 | 1 | 4 | 2 | 2 | 1 |
| Ps. aeruginosa ATCC 10145 | 4 | 2 | 8 | 4 | 8 | 2 |
| Citr. freundii ATCC 11606 | 1 | 0,25 | 0,5 | 0,5 | 1 | 0,25 |
| Morg. morganii ATCC 8019 | 1 | 0,25 | 0,5 | 0,5 | 1 | 0,25 |
| Proteus vulgaris ATCC 8427 | 1 | 1 | 0,25 | 1 | 1 | 0,25 |
| Kleb. pneumoniae ATCC 10031 | 1 | 0,25 | ≤0,03 | 0,5 | 1 | 0,25 |
| Sal. typhimurium ATCC 14028 | 1 | 0,25 | 4 | 1 | 1 | 0,5 |
| Sal. typhi ATCC 6539 | 1 | 0,25 | 0,5 | 0,5 | 1 | 0,25 |
| Escherichia coli ATCC 10799 | 1 | 0,5 | 0,25 | 0,5 | 1 | 0,5 |
| Escherichia coli ATCC 23559 | 0,5 | 0,25 | 0,25 | 0,5 | 1 | 0,25 |
| Ent. aerogenes ATCC 15038 | 1 | 0,25 | 0,5 | 0,5 | 1 | 0,25 |
| Ent. cloacae ATCC 23355 | 1 | 0,25 | 0,5 | 0,5 | 1 | 0,25 |
| Serr. marcescens ATCC 13880 | 1 | 0,5 | 2 | 1 | 2 | 0,5 |
| Shigella flexnerii ATCC 12022 | 1 | 0,25 | 0,25 | 0,12 | 1 | 0,25 |

| MICROORGANISMES | EXEMPLES | | | | | |
|---|---|---|---|---|---|---|
| | 15 | 16 | 18 | 20 | 22 | 23 |
| Bacillus subtilis ATCC 6633 | 0,06 | ≤0,03 | ≤0,03 | 0,12 | 0,12 | ≤0,03 |
| Bacillus cereus ATCC 11778 | 0,25 | 0,12 | 0,12 | 0,12 | 0,50 | 0,12 |
| Strep. faecalis ATCC 10541 | 1 | 0,12 | 1,0 | 2,0 | 2,0 | 0,25 |
| Staph. aureus ATCC 25178 | 0,25 | 0,12 | 0,12 | 0,12 | 0,5 | 0,12 |
| Staph. epidermidis ATCC 155-1 | 0,25 | 0,12 | 0,12 | 0,12 | 0,5 | 0,12 |
| Ps. aeruginosa ATCC 9721 | 2 | 0,5 | 1,0 | 2,0 | 2,0 | 0,50 |
| Ps. aeruginosa ATCC 10145 | 2 | 0,5 | 2,0 | 2,0 | 2,0 | 0,50 |
| Citr. freundii ATCC 11606 | 0,12 | 0,06 | 0,12 | 0,12 | 0,125 | ≤0,03 |
| Morg. morganii ATCC 8019 | 0,12 | 0,06 | 0,25 | 0,25 | 0,125 | ≤0,03 |
| Proteus vulgaris ATCC 8427 | 0,25 | 0,25 | 0,12 | 1,0 | 1,0 | 0,06 |
| Kleb. pneumoniae ATCC 10031 | 0,25 | 0,06 | 0,12 | ≤0,03 | ≤0,03 | ≤0,03 |
| Sal. typhimurium ATCC 14028 | 0,25 | 0,06 | 0,12 | 0,5 | 1,0 | 0,06 |
| Sal. typhi ATCC 6539 | 0,25 | ≤0,03 | 0,12 | 0,5 | 0,5 | ≤0,03 |
| Escherichia coli ATCC 10799 | 0,25 | 0,06 | 0,25 | 0,5 | 0,5 | 0,06 |
| Escherichia coli ATCC 23559 | 0,12 | ≤0,03 | 0,12 | 0,25 | 0,12 | ≤0,03 |
| Ent. aerogenes ATCC 15038 | 0,25 | 0,06 | 0,12 | 0,25 | 0,25 | ≤0,03 |
| Ent. cloacae ATCC 23355 | 0,25 | ≤0,03 | 0,12 | 0,25 | 0,12 | ≤0,03 |
| Serr. marcescens ATCC 13880 | 0,50 | 0,12 | 0,25 | 0,50 | 0,25 | 0,12 |
| Shigella flexnerii ATCC 12022 | 0,12 | ≤0,03 | 0,06 | 0,12 | 0,12 | ≤0,03 |

| MICROORGANISMES | EXEMPLES | | | |
|---|---|---|---|---|
| | 26 | 27 | 30 | 31 |
| Bacillus subtilis ATCC 6633 | 0,25 | 0,12 | 0,06 | 0,12 |
| Bacillus cereus ATCC 11778 | 0,50 | 0,25 | 0,12 | 0,12 |
| Strep. faecalis ATCC 10541 | 2,0 | 1,0 | 0,5 | 1,0 |
| Staph. aureus ATCC 25178 | 1,0 | 0,25 | 0,25 | 0,25 |
| Staph. epidermidis ATCC 155-1 | 0,5 | 0,25 | 0,25 | 0,5 |
| Ps. aeruginosa ATCC 9721 | 4,0 | 1,0 | 0,5 | 4,0 |
| Ps. aeruginosa ATCC 10145 | 4,0 | 2,0 | 1,0 | 4,0 |
| Citr. freundii ATCC 11606 | 1,0 | 0,25 | 0,12 | 0,25 |
| Morg. morganii ATCC 8019 | 1,0 | 0,25 | 0,12 | 0,5 |
| Proteus vulgaris ATCC 8427 | 1,0 | 0,25 | 0,12 | 0,25 |
| Kleb. pneumoniae ATCC 10031 | 0,25 | 0,25 | 0,25 | 0,5 |
| Sal. typhimurium ATCC 14028 | 1,0 | 0,5 | 0,25 | 0,5 |
| Sal. typhi ATCC 6539 | 1,0 | 0,5 | 0,25 | 0,5 |
| Escherichia coli ATCC 10799 | 1,0 | 0,5 | 0,25 | 0,5 |
| Escherichia coli ATCC 23559 | 0,5 | 0,25 | 0,12 | 0,12 |
| Ent. aerogenes ATCC 15038 | 1,0 | 0,25 | 0,25 | 0,5 |
| Ent. cloacae ATCC 23355 | 0,5 | 0,25 | 0,25 | 0,25 |
| Serr. marcescens ATCC 13880 | 2,0 | 0,5 | 0,5 | 1,0 |
| Shigella flexnerii ATCC 12022 | 0,5 | 0,25 | 0,06 | 0,25 |

| MICROORGANISMES | EXEMPLES | | | | | |
|---|---|---|---|---|---|---|
| | 33 | 34 | 35 | 36 | 37 | 38 |
| Bacillus subtilis ATCC 6633 | 0,25 | 0,25 | 0,25 | 0,12 | 0,06 | 0,06 |
| Bacillus cereus ATCC 11778 | 0,5 | 1 | 1 | 0,25 | 0,25 | 0,12 |
| Strep. faecalis ATCC 10541 | 0,25 | 0,25 | 2 | 0,12 | 1 | 0,5 |
| Staph. aureus ATCC 25178 | 0,25 | 0,5 | 1 | 0,25 | 0,25 | 0,12 |
| Staph. epidermidis ATCC 155-1 | 0,5 | 0,5 | 1 | 0,25 | 0,25 | 0,12 |
| Ps. aeruginosa ATCC 9721 | 4,0 | 2 | 4 | 2 | 2 | 1,0 |
| Ps. aeruginosa ATCC 10145 | 2,0 | 2 | 4 | 2 | 2 | 0,5 |
| Citr. freundii ATCC 11606 | 0,25 | 0,25 | 0,5 | 0,12 | 0,12 | 0,06 |
| Morg. morganii ATCC :019 | 0,5 | 0,25 | 0,5 | 0,25 | 0,25 | 0,06 |
| Proteus vulgaris ATCC 8427 | 0,5 | 1 | 1 | 0,25 | 0,5 | 0,06 |
| Kleb. pneumoniae ATCC 10031 | 0,5 | 0,5 | 0,5 | 0,12 | 0,25 | 0,12 |
| Sal. typhimurium ATCC 14028 | 0,5 | 1 | 0,5 | 0,25 | 0,25 | 0,12 |
| Sal. typhi ATCC 6539 | 0,25 | 0,5 | 0,5 | 0,25 | 0,12 | 0,12 |
| Escherichia coli ATCC 10799 | 0,5 | 1 | 1 | 0,25 | 0,25 | 0,12 |
| Escherichia coli ATCC 23559 | 0,25 | 0,5 | 0,5 | 0,12 | 0,12 | 0,06 |
| Ent. aerogenes ATCC 15038 | 0,5 | 0,5 | 1 | 0,25 | 0,25 | 0,12 |
| Ent. cloacae ATCC 23355 | 0,25 | 0,5 | 1 | 0,25 | 0,12 | 0,12 |
| Serr. marcescens ATCC 13880 | 1 | 1 | 1 | 1 | 1 | 0,25 |
| Shigella flexnerii ATCC 12022 | 0,25 | 0,25 | 0,5 | 0,12 | 0,06 | 0,06 |

| MICROORGANISMES | EXEMPLES | | | | | |
|---|---|---|---|---|---|---|
| | 39 | 40 | 41 | 42 | 43 | 44 |
| Bacillus subtilis ATCC 6633 | 0,25 | 0,06 | ≤0,03 | ≤0,03 | ≤0,03 | ≤0,03 |
| Bacillus cereus ATCC 11778 | 1 | 0,2 | 0,25 | 0,12 | 0,12 | 0,12 |
| Strep. faecalis ATCC 10541 | 4 | 2 | 1 | 1 | 0,12 | 0,12 |
| Staph. aureus ATCC 25178 | 1 | 0,12 | 0,25 | 0,12 | 0,12 | 0,12 |
| Staph. epidermidis ATCC 155-1 | 1 | 0,12 | 0,25 | 0,12 | 0,12 | 0,12 |
| Ps. aeruginosa ATCC 9721 | 2 | 2 | 1 | 2 | 0,5 | 0,5 |
| Ps. aeruginosa ATCC 10145 | 4 | 2 | 2 | 2 | 0,5 | 0,5 |
| Citr. freundii ATCC 11606 | 1 | 0,12 | 0,06 | 0,12 | 0,06 | 0,06 |
| Morg. morganii ATCC 8019 | 1 | 0,25 | 0,12 | 0,12 | 0,06 | 0,06 |
| Proteus vulgaris ATCC 8427 | 1 | 0,25 | 0,25 | 0,12 | 0,25 | 0,25 |
| Kleb. pneumoniae ATCC 10031 | 1 | 0,12 | 0,06 | ≤0,03 | 0,06 | 0,06 |
| Sal. typhimurium ATCC 14028 | 1 | 0,12 | 0,12 | 0,12 | 0,06 | 0,06 |
| Sal. typhi ATCC 6539 | 1 | 0,12 | 0,06 | 0,06 | ≤0,03 | ≤0,03 |
| Escherichia coli ATCC 10799 | 1 | 0,25 | 0,12 | 0,12 | 0,06 | 0,06 |
| Escherichia coli ATCC 23559 | 1 | 0,12 | 0,06 | 0,06 | ≤0,03 | ≤0,03 |
| Ent. aerogenes ATCC 15038 | 1 | 0,12 | 0,2 | 0,12 | 0,06 | 0,06 |
| Ent. cloacae ATCC 23355 | 1 | 0,12 | 0,06 | 0,06 | 0,03 | 0,03 |
| Serr. marcescens ATCC 13880 | 1 | 0,5 | 0,25 | 0,25 | 0,12 | 0,12 |
| Shigella flexnerii ATCC 12022 | 1 | 0,06 | 0,06 | 0,06 | ≤0,03 | ≤0,03 |

| MICROORGANISMES | EXEMPLES | | |
|---|---|---|---|
| | 45 | 46 | 47 |
| **Bacillus subtilis ATCC 6633** | 0,06 | ≤ 0,05 | < 0.03 |
| **Bacillus cereus ATCC 11778** | 0,25 | 0,12 | 0,12 |
| **Strep. faecalis ATCC 10541** | 1,0 | 1,0 | 0,5 |
| **Staph. aureus ATCC 25178** | 0,25 | 0,25 | 0,25 |
| **Staph. epidermidis ATCC 155-1** | 0,12 | 0,12 | 0,12 |
| **Ps. aeruginosa ATCC 9721** | 1,0 | 0,5 | 0,5 |
| **Ps. aeruginosa ATCC 10145** | 0,5 | 0,5 | 0.25 |
| **Citr. freundii ATCC 11606** | ≤ 0,03 | ≤ 0,03 | ≤ 0,03 |
| **Morg. morganii ATCC 8019** | 0,06 | ≤ 0,03 | ≤ 0,03 |
| **Proteus vulgaris ATCC 8427** | 0,5 | 0,25 | 0,12 |
| **Kleb. pneumoniae ATCC 10031** | ≤ 0,03 | ≤ 0,03 | ≤ 0,03 |
| **Sal. typhimurium ATCC 14028** | 0,06 | 0,06 | ≤ 0,03 |
| **Sal. typhi ATCC 6539** | ≤ 0,03 | ≤ 0,03 | ≤ 0.03 |
| **Escherichia coli ATCC 10799** | 0,06 | 0,06 | 0,06 |
| **Escherichia coli ATCC 23559** | ≤ 0,03 | ≤ 0,03 | ≤ 0,03 |
| **Ent. aerogenes ATCC 15038** | 0,06 | ≤ 0,03 | ≤ 0,03 |
| **Ent. cloacae ATCC 23355** | ≤ 0,03 | ≤ 0,03 | ≤ 0.03 |
| **Serr. marcescens ATCC 13880** | 0,12 | 0.12 | 0.12 |
| **Shigella flexnerii ATCC 12022** | < 0,03 | ≤ 0,03 | ≤ 0.03 |

Compte tenu de leurs bonnes propriétés pharmacologiques, les dérivés de formule générale I sont donc susceptibles d'être utilisés en médecine humaine et/ou vétérinaire, pour le traitement des infections aiguës, chroniques et récidivantes systémiques ou localisées, causées par des microorganismes Gram-positifs et Gram-négatifs sensibles aux produits objet de la présente invention, dans le tractus gastro-intestinal ou génito-urinaire, l'appareil respiratoire, la peau et les tissus mous, ainsi que les infections neurologiques et odonto-stomatologiques.

En thérapeutique humaine, la dose proposée des dérivés de la présente invention est environ comprise entre 400 et 1200 mg/jour pour un adulte, par exemple administrée sous la forme de comprimés ou de gélules. Cette posologie peut toutefois varier en fonction de la gravité de l'affection.

On indiquera ci-après, à titre d'exemples, deux formes galéniques particulières des dérivés, objet de la présente invention.

| Exemple de formule par comprimé | |
|---|---|
| Composé de l'exemple 2 | 0,400 g |
| Carboxyméthylamidon | 0,018 g |
| Polyvinylpyrrolidone K29-32 | 0,030 g |
| Cellulose microcristalline | 0,146 g |
| Silice colloïdale | 0,003 g |
| Stéarate de magnésium | 0,003 g |
| | 0,600 g |

| Exemple de formule par gélule | |
|---|---|
| Composé de l'exemple 16 | 0,400 g |
| Cellule microcristalline | 0,0356 g |
| Silice colloïdale | 0,0022 g |
| Stéarate de magnesium | 0,0022 g |
| | 0,440 g |

**Revendications**

1. Nouveaux composés hétérocycliques caractérisés en ce qu'ils répondent à la formule (I)

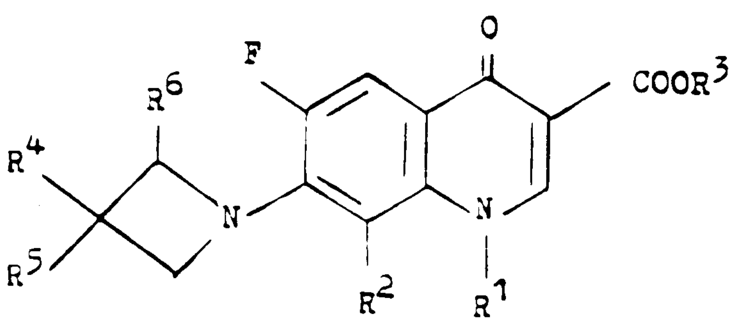

(I)

où $R^1$ représente un radical alkyle en $C_1$ à $C_4$, un radical halogénoalkyle en $C_1$ à $C_4$, un radical cyclopropyle, un radical aminoalkyle en $C_1$ à $C_4$, un radical phényle ou un radical phényle substitué par un ou plusieurs atome(s) de fluor,

$R^2$ représente un atome d'hydrogène, un atome d'halogène, ou bien $R^1$ et $R^2$ peuvent former ensemble une liaison représentée par un groupe X,

$R^3$ représente un atome d'hydrogène ou un radical alkyle inférieur en $C_1$ à $C_4$,

$R^4$ et $R^5$ et $R^6$ représentent indépendamment un atome d'hydrogène, un radical alkyle en $C_1$ à $C_4$, un radical hydroxyle, un radical amino, un radical aminoalkyle en $C_1$ à $C_4$, un radical alkyl en $C_1$ à $C_4$ amino, un radical dialkyl en $C_1$ à $C_4$ amino, un radical alkyl en $C_1$ à $C_4$ aminoalkyle en $C_1$ à $C_4$, un radical alcoxy en $C_1$ à $C_4$, un radical mésyloxy, un radical hydroxy-alkyle en $C_1$ à $C_4$, un radical cyano, un radical acylaminoalkyle en $C_1$ à $C_4$, un radical carboxylique, un radical carboxamido, un radical carboxyalkyle en $C_1$ à $C_4$, un atome d'halogène, un radical alkyl en $C_1$ à $C_4$ carboxylique, par exemple acétoxy, un radical acétamido ou un radical acétamidoalkyle en $C_1$ à $C_4$, le groupe alkyle en $C_1$ à $C_4$ libre terminal pouvant être fluoré dans ces deux derniers radicaux et l'atome d'azote dans le radical acétamidoalkyle en $C_1$ à $C_4$ pouvant être substitué par un groupe alkyle en $C_1$ à $C_4$.

X représente $-CH_2-CH_2-CHR^7-$, ou

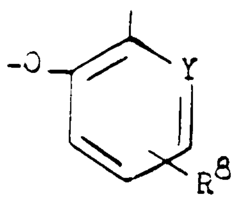

où $R^7$ représente un atome d'hydrogène ou un radical alkyle inférieur en $C_1$ à $C_4$
$R^8$ représente un atome d'hydrogène ou un atome d'halogène, et Y représente CH ou N,
à l'exception toutefois des composés de formule (I), dans laquelle :
$R^1$ représente un radical éthyle
$R^2$ représente un atome de fluor
$R^3$, $R^4$ et $R^6$ représentent un atome d'hydrogène, et
$R^5$ représente un radical éthylaminométhyle ($CH_3CH_2NHCH_2$).

2. Composés hétérocycliques selon la revendication 1, caractérisés en ce qu'ils répondent à la formule générale (I) avec $R^6$ et $R^3$ représenté par un atome d'hydrogène et $R^1$,$R^2$, $R^4$ et $R^5$ ayant la même signfication que précédemment.

3. Composés hétérocycliques selon les revendications 1 et 2, caractérisés en ce qu'ils répondent à la formule générale (Ia)

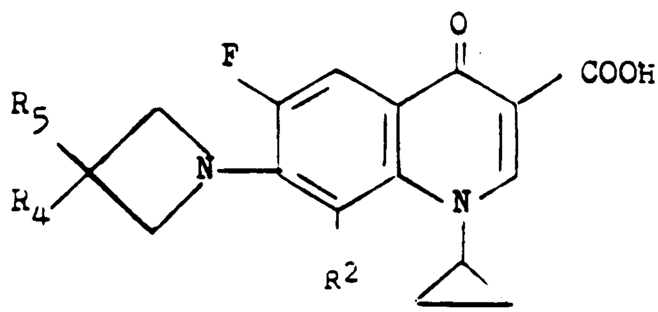

(Ia)

dans laquelle $R^2$, $R^4$ et $R^5$ ont la même significa tion que précédemment.

4. Les composés répondant à la formule générale I selon les revendications 1 et 2,sélectionnés dans le groupe suivant :
   . acide 1-(4-fluorophényl)-6,8-difluoro-7-(3-méthyl-3-trifluoroacétamido-1-azétidinyl)-1,4-dihydro-4-oxo-3-quinoléincarboxylique,
   . acide 1-(4-fluorophényl)-6,8-difluoro-7-(3 amino-3-méthyl-1-azétidinyl)-1,4-dihydro-4-oxo-3-quinoléincarboxylique,
   . acide 1-éthyl-6,8-difluoro-1,4-dihydro-4-oxo-7-(3-méthyl-3-trifluoroacétamido-1-azétidinyl)-3-quinoléincarboxylique,
   . acide 1-éthyl-6,8-difluoro-7-(3-amino-3-méthyl-1-azétidinyl)-1,4-dihydro-4-oxo-3-quinoléincarboxylique,
   . acide 1-éthyl-6-fluoro-7-(3-amino-3-méthyl-1-azétidinyl)-1,4-dihydro-4-oxo-3-quinoléincarboxylique,
   . acide 1-(2-fluoroéthyl)-6-fluoro-7-(3-méthyl-3-amino-1-azétidinyl)-1,4-dihydro-4-oxo-3-quinoléincarboxylique,
   . acide 1-(2,4-difluorophényl)-6-fluoro-7-(3-méthyl-3-amino-1-azétidinyl)-1,4-dihydro-4-oxo-3-quinoléincarboxylique,
   . acide 1-(2-fluoroéthyl)-6,8-difluoro-7-(3-amino-3-méthyl-1-azétidinyl)-1,4-dihydro-4-oxo-3-quinoléincarboxylique,
   . acide 1-(4-fluorophényl)-6-fluoro-7-(3-amino-3-méthyl-1-azétidinyl)-1,4-dihydro-4-oxo-3-quinoléin-

carboxylique,

. acide 1-(2,4-difluorophényl)-6,8-difluoro-7-(3-méthyl-3-trifluoroacétamido-1-azétidinyl)-1,4-dihydro-4-oxo-3-quinoléincarboxylique,

. acide 1-(2,4-difluorophényl)-6,8-difluoro-7-(3-amino-3-méthyl-1-azétidinyl)-1,4-dihydro-4-oxo-3-quinoléincarboxylique.

**5.** Les composés répondant à la formule générale I, selon les revendications 1,2 et 3, sélectionnés dans le groupe suivant :

. 1-cyclopropyl-6,8-difluoro-7-(3-hydroxy-1-azétidinyl)-1,4-dihydro-4-oxo 2- quinoléincarboxylate d'éthyle,

. acide 1-cyclopropyl-6,8-difluoro-7)(3-hydroxy-1-azétidinyl)-1,4-dihydro-4-oxo-3-quinoléincarboxylique,

. 1-cyclopropyl-6,8-difluoro-7-(3-mésyloxy-1-azétidinyl)-1,4-dihydro-4-oxo-3-quinoléincarboxylate d'éthyle,

. 7-(3-acétylaminométhyl-1-azétidinyl)-1-cyclopropyl-6,8-difluoro-1,4-dihydro-4-oxo-3-quinoléincarboxylate d'éthyle,

. acide 1-cyclopropyl-6,8-difluoro-7-(1-azétidinyl-3-carboxylique)-1,4-dihydro-4-oxo-3-quinoléincarboxylique,

. acide 1-cyclopropyl-6,8-difluoro-7-(1-azétidinyl-3-carboxamide)-1,4-dihydro-4-oxo-3-quinoléincarboxylique,

. acide 1-cyclopropyl-6,8-difluoro-7-(1-azétidinyl-3-cyano)-1,4-dihydro-4 oxo-3-quinoléincarboxylique,

. acide 1-cyclopropyl-6,8-difluoro-7-(1-azétidinyl-3-méthyl-3-hydroxy)-1,4-dihydro-4-oxo-3-quinoléincarboxylique,

. acide 7-(3-trifluoroacétamidométhyl -1-azétidinyl)-1-cyclopropyl-6,8-difluoro-1,4-dihydro-4-oxo-3-quinoléincarboxylique,

. acide 7-(3-aminométhyl-1-azétidinyl)-1-cyclopropyl-6,8-difluoro-1,4-dihydro-4-oxo-3-quinoléincarboxylique,

. acide 1-cyclopropyl-6,8-difluoro-7-(3-mésyloxy-1-azétidinyl)-1,4-dihydro-4-oxo-3-quinoléincarboxylique,

. acide 7-[3-(N'-éthyl-trifluoroacétamidométhyl)-1-azétidinyl]-6,8-difluoro-1,4-dihydro-1-cyclopropyl-4-oxo-3-quinoléincarboxylique,

. acide 7-(3-éthylaminométhyl-1-azétidinyl)-6,8-difluoro-1,4-dihydro-1-cyclopropyl-4-oxo-3-quinoléincarboxylique,

. acide 1-cyclopropyl-6,8-difluoro-1,4-dihydro-7-(1-azétidinyl)-4-oxo-3-quinoléincarboxylique,

. acide 1-cyclopropyl-6,8-difluoro-7-(3-méthyl-3-trifluoroacétamido-1-azétidinyl)-4-oxo-3-quinoléincarboxylique,

. acide 1-cyclopropyl-6,8-difluoro-1,4-dihydro-7-(3-méthyl-3-amino-1-azétidinyl)-4-oxo-3-quinoléincarboxylique,

. acide -cyclopropyl-6,8-difluoro-7-(3-acétoxy-1-azétidinyl)-1,4-dihydro-4-oxo-3-quinoléincarboxylique,

. acide 1-cyclopropyl-6-fluoro-7-(3-hydroxy-1-azétidinyl-1,4-dihydro-4-oxo-3-quinoléincarboxylique,

. acide 1-cyclopropyl-6,8-difluoro-7-(3-amino-1-azétidinyl)-1,4-dihydro-4-oxo-3-quinoléincarboxylique,

. acide 1-cyclopropyl-6,8-difluoro-7-(3-trifluoroacétamidométhyl-3-méthyl-1-azétidinyl)-1,4-dihydro-4-oxo-3-quinoléincarboxylique,

. acide 1-cyclopropyl-6,8-difluoro-7-(3-N'-éthyl-trifluoroacétamidométhyl-3-méthyl-1-azétidinyl)-1,4-dihydro-4-oxo-3-quinoléincarboxylique,

. acide 1-cyclopropyl-6,8-difluoro-1,4-dihydro-4-oxo-7-(3-aminométhyl-3-méthyl-1-azétidinyl)-3-quinoléincarboxylique,

. acide 1-cyclopropyl-6,8-difluoro-7-(3-éthylaminométhyl-3-méthyl-1-azétidinyl)-1,4-dihydro-4-oxo-3-quinoléincarboxylique,

. acide 1-cyclopropyl-6-fluoro-7-(3-trifluoroacétamidométhyl-3-méthyl-1-azétidinyl)-1,4-dihydro-4-oxo-3-quinoléincarboxylique,

. acide 1-cyclopropyl-6-fluoro-7-(3-trifluoroacétamidoéthylaminométhyl-3-méthyl-1-azétidinyl)-1,4-dihydro-4-oxo-3-quinoléincarboxylique,

. acide 1-cyclopropyl-6-fluoro-1,4-dihydro-4-oxo-7-(3-aminométhyl-3-méthyl-1-azétidinyl)-3-quinoléincarboxylique,

. acide 1-cyclopropyl-6-fluoro-7-(3-éthylaminométhyl-3-méthyl-1-azétidinyl)-1,4-dihydro-4-oxo-3-quinoléincarboxylique,
. acide 1-cyclopropyl-6,8-difluoro-1,4-dihydro-4-oxo-7-(3-méthyl-3-N,N-diméthylamino-1-azétidinyl)-3-quinoléincarboxylique,
. acide 1-cyclopropyl-6-fluoro-7-(3-amino-3-méthyl-1-azétidinyl) -1,4-dihydro-4-oxo-3-quinoléincarboxylique,
. acide 1-cyclopropyl-6,8-difluoro-7-(3-diméthylamino-1-azétidinyl)-1,4-dihydro-4-oxo-3-quinoléincarboxylique,
. acide 1-cyclopropyl-6-fluoro-7-(3-diméthylamino-1-azétidinyl)-1,4-dihydro-4-oxo-3-quinoléincarboxylique,
. chlorhydrate de l'acide 1-cyclopropyl-6,8-difluoro-7-(3-amino -3-méthyl-1-azétidinyl)-1,4-dihydro-4-oxo-3-quinoléincarboxylique,
. sel de sodium acide 1-cyclopropyl-6,8-difluoro-7-(3-amino-3-méthyl-1-azétidinyl)-1,4-dihydro-4-oxo-3-quinoléincarboxylique.
. acide 1-cyclopropyl-6,8-difluoro-7-(3-methylamino-1-azétidinyl)-1,4-dihydro-4-oxo-3-quinoléincarboxylique,
. acide 1-cyclopropyl-6-fluoro-7-(3-méthylamino-1- azétidinyl)-1,4-dihydro-4-oxo-3-quinoléincarboxylique,
. acide 1-cyclopropyl-6-fluoro-7-(3-amino-1-azétidinyl)- 1,4-dihydro-4-oxo-3-quinoléincarboxylique,

**6.** Procédé de préparation des dérivés de formule I, selon les revendications 1 à 5, caractérisé en ce qu'il consiste à faire réagir un composé hétérocyclique de formule (II)

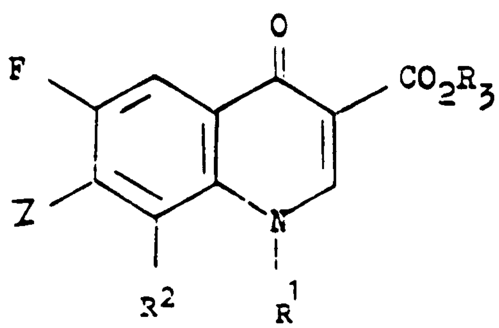

II .

où $R^1$, $R^2$ et $R^3$ ont la même signification que précédemment et Z représente un atome d'halogène ; avec un composé représenté par la formule (III)

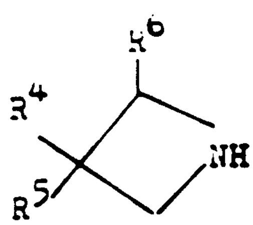

(III)

où $R^4$ et $R^5$ et $R^6$ ont la même signification que précédemment.

**7.** Procédé de préparation des dérivés de formule I selon les revendications 1 à 5, caractérisé en ce qu'il consiste à faire réagir un composé hétérocyclique de formule (II), où $R^1$, $R^2$ et $R^3$ ont la même signification que précédemment, et Z représente un radical amino, avec un composé représenté par la formule (IV)

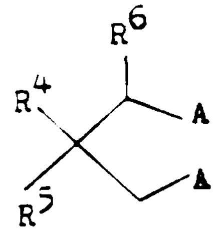

(IV)

où $R^4$ et $R^5$ et $R^6$ ont la même signification que précédemment, et A représente un atome d'halogène, un radical hydroxyle, un radical alkylsulfonyloxy inférieur en $C_1$ à $C_4$ ou un radical phényl-sulfonyloxy.

**8.** A titre de médicaments, les composés de formule générale (I) et leurs sels thérapeutiquement acceptables selon l'une des revendications 1 à 5 spécialement comme antibactériens.

**9.** Compositions pharmaceutiques, caractérisées par le fait qu'elles contiennent, outre un support pharmaceutiquement acceptable, au moins un composé de formule générale (I) ou l'un de leurs sels physiologiquement acceptables, selon l'une des revendications 1 à 5.

## Claims

**1.** New heterocyclic compounds characterised in that they correspond to formula I

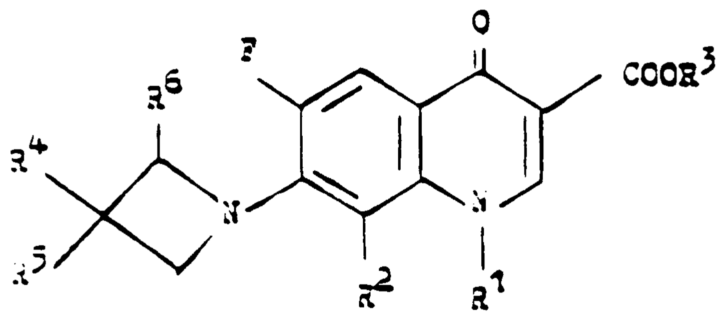

(I)

where

. $R^1$ represents a $C_1$ to $C_4$ alkyl radical, a $C_1$ to $C_4$ haloalkyl radical, a cyclopropyl radical, a $C_1$ to $C_4$ amino-alkyl radical, a phenyl radical or a phenyl radical, substituted with one or more fluorine atoms;

. $R^2$ represents a hydrogen atom, a halogen atom, or $R^1$ and $R^2$ may together form an X group;

. $R^3$ represents a hydrogen atom or a $C_1$ to $C_4$ alkyl radical;

. $R^4$ and $R^5$ and $R^6$ independently represent a hydrogen atom, a $C_1$ to $C_4$ alkyl radical, a hydroxyl radical, and amino radical, a $C_1$ to $C_4$ dialkylamino radical, a $C_1$ to $C_4$ alkylaminoalkyl radical, a $C_1$ to $C_4$ alkoxy radical,

a mesyloxy radical, a hydroxyalkyl radical, a cyano radical, a $C_1$ to $C_4$ acylaminoalkyl radical, a carboxyl radical, a halogen atom, a $C_1$ to $C_4$ alkylcarboxy radical e.g. acetoxy, an acetamido radical or $C_1$ to $C_4$ acetamidoalkyl radical in these last two radicals, the terminal free alky group may be fluorinated and the nitrogen atom in the acetamidoalkyl radical may carry a $C_1$ to $C_4$ alkyl substituent;

. X represents $-CH-CH_2-CHR^7-$, or

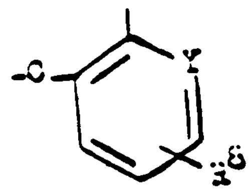

where

. $R^7$ represents a hydrogen atom or a $C_1$ to $C_4$ alkyl radical,

. $R^8$ represents a hydrogen atom or a halogen atom, and Y represents CH or N,

with the exception however of compounds of formula (I), in which:

$R^1$ represents an ethyl radical

$R^2$ represents a fluorine atom

$R^3$, $R^4$ and $R^6$ represents hydrogen atoms, and

$R^5$ represents an ethylaminomethyl radical ($CH_3CH_2NHCH_2$).

**2.** Heterocyclic compounds according to Claim 1, characterised in that they correspond to general formula (I) with $R^6$ and $R^3$ represented by a hydrogen atom and $R^1$,$R^2$, $R^4$ and $R^5$ having the same meaning as hereinbefore.

**3.** Heterocyclic compounds according to Claims 1 and 2, characterised in that they correspond to general formula (Ia)

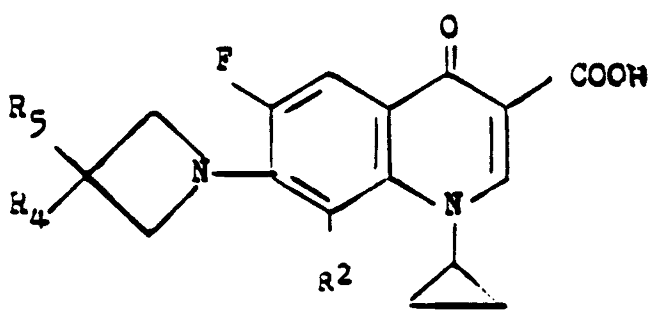

(Ia)

in which $R^2$, $R^4$ and $R^5$ have the same meaning as hereinfore.

**4.** Compounds corresponding to general formula (I) according to Claims 1 and 2, selected from the following group:

. 1-(4-fluorophenyl)-6,8-difluoro-7-(3-methyl-3-trifluoroacetamido-1-azetidinyl)-1,4-dihydro-4-oxo-3-quinolinecarboxylic acid,

. 1-(4-fluorophenyl)-6,8-difluoro-7-(3-amino-3-methyl-1-azetidinyl)-1,4-dihydro-4-oxo-3-quinolinecarboxylic acid.

. 1-ethyl-6,8-difluoro-1,4-dihydro-4-oxo-7-(3-methyl-3-trifluoroacetamido-1-azetidinyl)-3-quinolinecarboxylic acid,

. 1-ethyl-6,8-difluoro-7-(3-amino-3-methyl-1-azetidinyl)-1,4-dihydro-4-oxo-3-quinolinecarboxylic acid,

. 1-ethyl-6-fluoro-7-(3-amino-3-methyl-1-azetidinyl)-1,4-dihydro-4-oxo-3-quinolinecarboxylic acid,

. 1-(2-fluoroethyl)-6-fluoro-7-(3-methyl-3-amino-1-azetidinyl)-1,4-dihydro-4-oxo-3-quinolinecarboxylic acid,

. 1-(2,4-difluorophenyl)-6-fluoro-7-(3-methyl-3-amino-1-azetidinyl)-1,4-dihydro-4-oxo-3-quinolinecarboxylic acid,

. 1-(2-fluoroethyl)-6,8-difluoro-7-(3-amino-3-methyl-1-azetidinyl)-1,4-dihydro-4-oxo-3-quinolinecarboxylic acid,

. 1-(4-fluorophenyl)-6-fluoro-7-(3-amino-3-methyl-1-azetidinyl)-1,4-dihydro-4-oxo-3-quinolinecarboxylic acid,

. 1-(2,4-difluorophenyl)-6,8-difluoro-7-(3-methyl-3-trifluoroacetamido-1-azetidinyl)-1,4-dihydro-4-oxo-3-quinolinecarboxylic acid,

. 1-(2,4-difluorophenyl)-6,8-difluoro-7-(3-amino-3-methyl-1-azetidinyl)-1,4-dihydro-4-oxo-3-quinolinecarboxylic acid.

**5.** Compounds corresponding to general formula I, according to Claims 1, 2 and 3, selected from the following groups

. ethyl 1-cyclopropyl-6,8-difluoro-7-(3-hydroxy-1-azetidinyl)-1,4-dihydro-4-oxo-3-quinolinecarboxylate,
. 1-cyclopropyl-6,8-difluoro-7-(3-hydroxy-1-azetidinyl)-1,4-dihydro-4-oxo-3-quinolinecarboxylic acid,
. ethyl 1-cyclopropyl-6,8-difluoro-7-(3-mesyloxy-1-azetidinyl)-1,4-dihydro-4-oxo-3-quinolinecarboxylate,
. ethyl 7-(3-acetylaminomethyl-1-azetidinyl)-1-cyclopropyl-6,8-difluoro-1,4-dihydro-4-oxo-3-quinolinecarboxylate,
. 1-cyclopropyl-6,8-difluoro-7-(3-carboxy-1-azetidinyl)-1,4-dihydro-4-oxo-3-quinolinecarboxylic acid,
. 1-cyclopropyl-6,8-difluoro-7-(3-carbamoyl-1-azetidinyl)-1,4-dihydro-4-oxo-3-quinolinecarboxylic acid,
. 1-cyclopropyl-6,8-difluoro-7-(3-cyano-1-azetidinyl)-1,4-dihydro-4-oxo-3-quinolinecarboxylic acid,
. 1-cyclopropyl-6,8-difluoro-7-(3-methyl-3-hydroxy-1-azetidinyl)-1,4-dihydro-4-oxo-3-quinolinecarboxylic acid,
. 7-(3-trifluoroacetamidomethyl-1-azetidinyl)-1-cyclopropyl-6,8-difluoro-1,4-dihydro-4-oxo-3-quinolinecarboxylic acid,
. 7-(3-aminomethyl-1-azetidinyl)-1-cyclopropyl-6,8-difluoro-1,4-dihydro-4-oxo-3-quinolinecarboxylic acid,
. 1-cyclopropyl-6,8-difluoro-7-(3-mesyloxy-1-azetidinyl)-1,4-dihydro-4-oxo-3-quinolinecarboxylic acid,
. 7-[3-(N'-ethyl-N'-trifluoroacetamidomethyl)-1-azetidinyl]-6,8-difluoro-1,4-dihydro-1-cyclopropyl-4-oxo-3-quinolinecarboxylic acid,
. 7-(3-N'-ethylaminomethyl-1-azetidinyl)-6,8-difluoro-1,4-dihydro-1-cyclopropyl-4-oxo-3-quinolinecarboxylic acid,
. 1-cyclopropyl-6,8-difluoro-1,4-dihydro-7-(1-azetidinyl)-4-oxo-3-quinolinecarboxylic acid,
. 1-cyclopropyl-6,8-difluoro-7-(3-methyl-3-trifluoroacetamido-1-azetidinyl)-4-oxo-3-quinolinecarboxylic acid,
. 1-cyclopropyl-6,8-difluoro-1,4-dihydro-7-(3-methyl-3-amino-1-azetidinyl)-4-oxo-3-quinolinecarboxylic acid,
. 1-cyclopropyl-6,8-difluoro-7-(3-acetoxy-1-azetidinyl)-1,4-dihydro-4-oxo-3-quinolinecarboxylic acid,
. 1-cyclopropyl-6-fluoro-7-(3-hydroxy-1-azetidinyl)-1,4-dihydro-4-oxo-3-quinolinecarboxylic acid,
. 1-cyclopropyl-6,8-difluoro-7-(3-amino-1-azetidinyl)-1,4-dihydro-4-oxo-3-quinolinecarboxylic acid,
. 1-cyclopropyl-6,8-difluoro-7-(3-trifluoroacetamidomethyl-3-methyl-1-azetidinyl)-1,4-dihydro-4-oxo-3-quinolinecarboxylic acid,
. 1-cyclopropyl-6,8-difluoro-7-(3-N-ethyl-trifluoroacetamidomethyl-3-methyl-1-azetidinyl)-1,4-dihydro-4-oxo-3-quinolinecarboxylic acid,
. 1-cyclopropyl-6,8-difluoro-1,4-dihydro-4-oxo-7-(3-aminomethyl-3-methyl-1-azetidinyl)-3-quinolinecarboxylic acid,
. 1-cyclopropyl-6,8-difluoro-7-(3-ethylaminomethyl-3-methyl-1-azetidinyl)-1,4-dihydro-4-oxo-3-quinolinecarboxylic acid,
. 1-cyclopropyl-6-fluoro-7-(3-trifluroacetamidomethyl-3-methyl-1-azetidinyl)-1,4-dihydro-4-oxo-3-quinolinecarboxylic acid,
. 1-cyclopropyl-6-fluoro-7-(3-trifluoroacetamidoethylaminomethyl-3-methyl-1-azetidinyl)-1,4-dihydro-4-oxo-3-quinolinecarboxylic acid,
. 1-cyclopropyl-6-fluoro-1,4-dihydro-4-oxo-7-(3-aminomethyl-3-methyl-1-azetidinyl)-3-quinolinecarboxylic acid,
. 1-cyclopropyl-6-fluoro-7-(3-ethylaminomethyl-3-methyl-1-azetidinyl)-1,4-dihydro-4-oxo-3-quinolinecarboxylic acid,
. 1-cyclopropyl-6,8-difluoro-1,4-dihydro-4-oxo-7-(3-methyl-3-N,N-dimethylamino-1-azetidinyl)-3-quinolinecarboxylic acid,
. 1-cyclopropyl-6-fluoro-7-(3-amino-3-methyl-1-azetidinyl)-1,4-dihydro-4-oxo-3-quinolinecarboxylic acid,
. 1-cyclopropyl-6,8-difluoro-7-(3-dimethylamino-1-azetidinyl)-1,4-dihydro-4-oxo-3-quinolinecarboxylic acid,

- . 1-cyclopropyl-6-fluoro-7-(3-dimethylamino-1-azetidinyl)-1,4-dihydro-4-oxo-3-quinolinecarboxylic acid
- . 1-cyclopropyl-6,8-difluoro-7-(3-amino-3-methyl-1-azetidinyl)-1,4-dihydro-4-oxo-3-quinolinecarboxylic acid hydrochloride
- . Sodium salt of 1-cyclopropyl-6,8-difluoro-7-(3-amino-3-methyl-1-azetidinyl)-1,4-dihydro-4-oxo-3-quinolinecarboxylic acid.
- . 1 - cyclopropyl-6,8-difluoro-7-(3-methylamino-1-azetidinyl 1,4-dihydro-4-oxo-3-quinolinecarboxylic acid,
- . 1 - cyclopropyl-6-fluoro-7-(3-methylamino-1-azetidinyl)-1,4-dihydro-4-oxo-3-quinolinecarboxylic acid,
- . 1 - cyclopropyl-6-fluoro-7-(3-amino-1-azetidinyl)-1,4-dihydro-4-oxo-3-quinolinecarboxylic acid.

**6.** Process for preparing derivatives or formula I, according to Claims 1 to 5, characterised in that it comprises reacting a heterocyclic compound of formula (II)

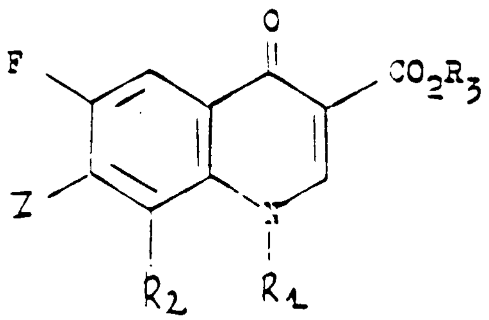

II

where $R^1$, $R^2$ and $R^3$ have the same meaning as hereinbefore and Z represents a halogen atom; with a compound represented by formula (III)

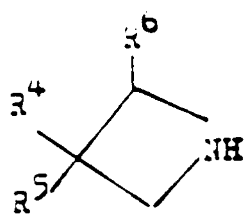

(III)

where $R^4$ and $R^5$ and $R^6$ have the same meaning as hereinbefore.

**7.** Process for preparing derivatives of formula I according to Claims 1 to 5, characterised in that it comprises reacting a heterocyclic compound of formula II, where $R^1$, $R^2$ and $R^3$ have the same meaning as hereinbefore, and Z represents an amino radical, with a compound represented by formula (IV)

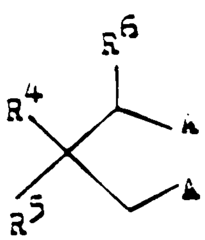

(IV)

where $R^4$ and $R^5$ and $R^6$ have the same meaning as hereinbefore, and A represents a halogen atom, a hydroxyl radical, a $C_1$ to $C_4$ alkylsulphonyloxy radical or phenyl sulphonyloxy radical.

8. By way of medicines, the compounds of general formula (I) and their therapeutically acceptable salts according to one of claims 1 to 5 especially for use as antibacterial agents.

9. Pharmaceutical compositions, characterised by the fact that they contain, besides an acceptable pharmaceutical support, at least one compound of general formula (I) or a physiologically acceptable salt of such a compound, according to claims 1 to 5.

**Patentansprüche**

1. Neue heterocyclische Verbindungen, dadurch gekennzeichnet, daß sie der allgemeinen Formel I

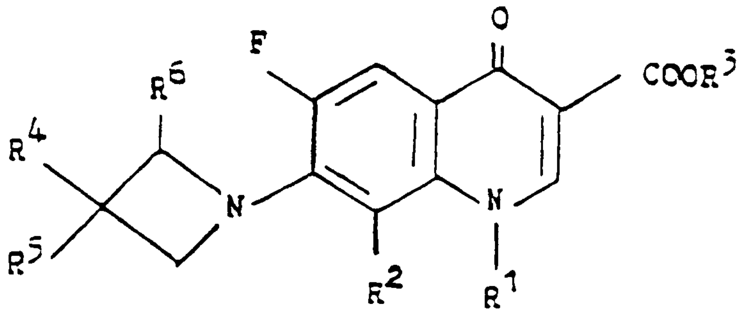

(I)

entsprechen, in der $R^1$ ein Alkyl-Radikal mit 1 bis 4 Kohlenstoffatomen, ein Halogenalkyl-Radikal mit 1 bis 4 Kohlenstoffatomen, ein Cyclopropyl-Radikal, ein Aminoalkyl-Radikal mit 1 bis 4 Kohlenstoffatomen, ein Phenyl-Radikal oder ein durch ein oder mehrere Fluoratome substituiertes Phenyl-Radikal darstellt,

$R^2$ ein Wasserstoffatom oder ein Halogenatom bedeutet, oder $R^1$ und $R^2$ auch zusammen eine durch eine Gruppe X dargestellte Bindung bilden können,

$R^3$ ein Wasserstoffatom oder ein niederes Alkyl-Radikal mit 1 bis 4 Kohlenstoffatomen ist,

$R^4$, $R^5$ und $R^6$ unabhängig voneinander ein Wasserstoffatom, ein Alkyl-Radikal mit 1 bis 4 Kohlenstoffatomen, ein Hydroxyl-Radikal, ein Amino-Radikal, ein Aminoalkyl-Radikal mit 1 bis 4 Kohlenstoffatomen, ein Alkylamino-Radikal mit 1 bis 4 Kohlenstoffatomen, ein Dialkylamino-Radikal mit 1 bis 4 Kohlenstoffatomen, ein Alkylaminoalkyl-Radikal mit 1 bis 4 Kohlenstoffatomen, ein Alkoxy-Radikal mit 1 bis 4 Kohlenstoffatomen, ein Mesyloxy-Radikal, ein Hydroxyalkyl-Radikal mit 1 bis 4 Kohlenstoffatomen, ein Cyano-Radikal, ein Acylaminoalkyl-Radikal mit 1 bis 4 Kohlenstoffatomen, ein Carboxyl-Radikal, ein Carboxamido-Radikal, ein Carboxalkyl-Radikal mit 1 bis 4 Kohlenstoffatomen, ein Halogenatom, ein Alkylcarboxyl-Radikal mit 1 bis 4 Kohlenstoffatomen, beispielsweise Acetoxy, ein Acetamido-Radikal oder ein Acetamidoalkyl-Radikal mit 1 bis 4 Kohlenstoffatomen darstellen, wobei die endständige freie Alkyl-Gruppe mit 1 bis 4 Kohlenstoffatomen in den zwei letzten Radikalen fluoriert und das Stickstoffatom in dem Acetamido-Radikal mit 1 bis 4 Kohlenstoffatomen durch eine Alkyl-Gruppe mit 1 bis 4 Kohlenstoffatomen substituiert sein können,

X die Gruppe $-CH_2-CH_2-CHR^7-$ oder

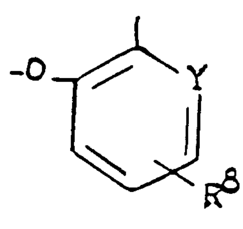

bedeutet, worin $R^7$ ein Wasserstoffatom oder ein niederes Alkyl-Radikal mit 1 bis 4 Kohlenstoffatomen ist,

$R^8$ ein Wasserstoffatom oder ein Halogenatom darstellt, und

Y CH oder N bedeutet, jedoch mit der Ausnahme der Verbindungen der Formel I, in der

$R^1$ ein Ethyl-Radikal darstellt,

$R^2$ ein Fluoratom bedeutet,
$R^3$, $R^4$ und $R^6$ Wasserstoffatome sind, und
$R^5$ ein Ethylaminomethyl-Radikal ($CH_3CH_2NHCH_2$) darstellt.

**2.** Heterocyclische Verbindungen nach Anspruch 1, dadurch gekennzeichnet, daß sie der allgemeinen Formel I entsprechen, worin $R^6$ und $R^3$ ein Wasserstoffatom darstellen und $R^1$, $R^2$, $R^4$ und $R^5$ die vorstehend angegebene Bedeutung besitzen.

**3.** Heterocyclische Verbindungen nach Anspruch 1 und 2, dadurch gekennzeichnet, daß sie der allgemeinen Formel Ia

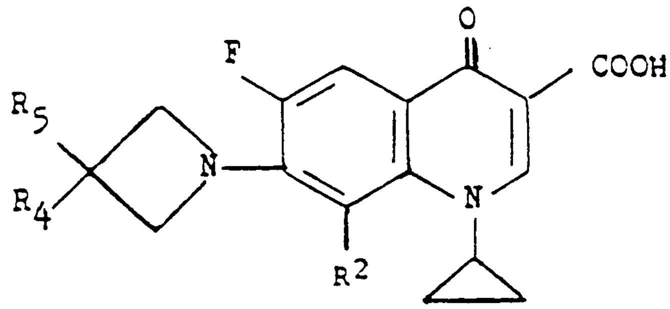

(Ia)

entsprechen, worin $R^2$, $R^4$ und $R^5$ die vorstehend angegebene Bedeutung besitzen.

**4.** Verbindungen der allgemeinen Formel I gemäß den Ansprüchen 1 und 2, ausgewählt aus der folgenden Gruppe:
. 1-(4-Fluorphenyl)-6,8-difluor-7-(3-methyl-3-trifluoracetamido-1-azetidinyl)-1,4-dihydro-4-oxo-3-chinolincarbonsäure,
. 1-(4-Fluorphenyl)-6,8-difluor-7-(3-amino-3-methyl-1-azetidinyl)-1,4-dihydro-4-oxo-3-chinolincarbonsäure,
. 1-Ethyl-6,8-difluor-7-(3-methyl-3-trifluoracetamido-1-azetidinyl)-1,4-dihydro-4-oxo-3-chinolincarbonsäure,
. 1-Ethyl-6,8-difluor-7-(3-amino-3-methyl-1-azetidinyl)-1,4-dihydro-4-oxo-3-chinolincarbonsäure,
. 1-Ethyl-6-fluor-7-(3-amino-3-methyl-1-azetidinyl)-1,4-dihydro-4-oxo-3-chinolincarbonsäure,
. 1-(2-Fluorethyl)-6-fluor-7-(3-amino-3-methyl-1-azetidinyl)-1,4-dihydro-4-oxo-3-chinolincarbonsäure,
. 1-(2,4-Difluorphenyl)-6-fluor-7-(3-amino-3-methyl-1-azetidinyl)-1,4-dihydro-4-oxo-3-chinolincarbonsäure,
. 1-(2-Fluorethyl)-6,8-difluor-7-(3-amino-3-methyl-1-azetidinyl)-1,4-dihydro-4-oxo-3-chinolincarbonsäure,
. 1-(4-Fluorphenyl)-6-fluor-7-(3-amino-3-methyl-1-azetidinyl)-1,4-dihydro-4-oxo-3-chinolincarbonsäure,
. 1-(2,4-Difluorphenyl)-6,8-difluor-7-(3-methyl-3-trifluoracetamido-1-azetidinyl)-1,4-dihydro-4-oxo-3-chinolincarbonsäure,
. 1-(2,4-Difluorphenyl)-6,8-difluor-7-(3-amino-3-methyl-1-azetidinyl)-1,4-dihydro-4-oxo-3-chinolincarbonsäure.

**5.** Verbindungen der allgemeinen Formel I gemäß den Ansprüchen 1, 2 und 3, ausgewählt aus der folgenden Gruppe:
. 1-Cyclopropyl-6,8-difluor-7-(3-hydroxy-1-azetidinyl)-1,4-dihydro-4-oxo-2-chinolincarhonsäure-ethylester,
. 1-Cyclopropyl-6,8-difluor-7-(3-hydroxy-1-azetidinyl)-1,4-dihydro-4-oxo-3-chinolincarbonsäure,
. 1-Cyclopropyl-6,8-difluor-7-(3-mesyloxy-1-azetidinyl)-1,4-dihydro-4-oxo-3-chinolincarbonsäure-ethylester,
. 1-Cyclopropyl-6,8-difluor-7-(3-acetylaminomethyl-1-azetidinyl)-1,4-dihydro-4-oxo-3-chinolincarbonsäure-ethylester,
. 1-Cyclopropyl-6,8-difluor-7-(3-carboxyl-1-azetidinyl)-1,4-dihydro-4-oxo-3-chinolincarbonsäure,
. 1-Cyclopropyl-6,8-difluor-7-(3-carboxamido-1-azetidinyl)-1,4-dihydro-4-oxo-3-chinolincarbonsäure,

. 1-Cyclopropyl-6,8-difluor-7-(3-cyano-1-azetidinyl)-1,4-dihydro-4-oxo-3-chinolincarbonsäure,
. 1-Cyclopropyl-6,8-difluor-7-(3-methyl-3-hydroxy-1-azetidinyl)-1,4-dihydro-4-oxo-3-chinolincarbonsäure,
. 1-Cyclopropyl-6,8-difluor-7-(3-trifluoracetamidomethyl-1-azetidinyl)-1,4-dihydro-4-oxo-3-chinolincarbonsäure,
. 1-Cyclopropyl-6,8-difluor-7-(3-aminomethyl-1-azetidinyl)-1,4-dihydro-4-oxo-3-chinolincarbonsäure,
. 1-Cyclopropyl-6,8-difluor-7-(3-mesyloxy-1-azetidinyl)-1,4-dihydro-4-oxo-3-chinolincarbonsäure,
. 1-Cyclopropyl-6,8-difluor-7-[3-(N'-ethyl-trifluoracetamidomethyl)-1-azetidinyl]-1,4-dihydro-4-oxo-3-chinolincarbonsäure,
. 1-Cyclopropyl-6,8-difluor-7-(3-ethylaminomethyl-1-azetidinyl)-1,4-dihydro-4-oxo-3-chinolincarbonsäure,
. 1-Cyclopropyl-6,8-difluor-7-(1-azetidinyl)-1,4-dihydro-4-oxo-3-chinolincarbonsäure,
. 1-Cyclopropyl-6,8-difluor-7-(3-methyl-3-trifluoracetamido-1-azetidinyl)-4-oxo-3-chinolincarbonsäure,
. 1-Cyclopropyl-6,8-difluor-7-(3-amino-3-methyl-1-azetidinyl)-1,4-dihydro-4-oxo-3-chinolincarbonsäure,
. 1-Cyclopropyl-6,8-difluor-7-(3-acetoxy-1-azetidinyl)-1,4-dihydro-4-oxo-3-chinolincarbonsäure,
. 1-Cyclopropyl-6-fluor-7-(3-hydroxy-1-azetidinyl)-1,4-dihydro-4-oxo-3-chinolincarbonsäure,
. 1-Cyclopropyl-6,8-difluor-7-(3-amino-1-azetidinyl)-1,4-dihydro-4-oxo-3-chinolincarbonsäure,
. 1-Cyclopropyl-6,8-difluor-7-(3-trifluoracetamidomethyl-3-methyl-1-azetidinyl)-1,4-dihydro-4-oxo-3-chinolincarbonsäure,
. 1-Cyclopropyl-6,8-difluor-7-(3-N'-ethyl-trifluoracetamidomethyl-3-methyl-1-azetidinyl)-1,4-dihydro-4-oxo-3-chinolincarbonsäure,
. 1-Cyclopropyl-6,8-difluor-7-(3-aminomethyl-3-methyl-1-azetidinyl)-1,4-dihydro-4-oxo-3-chinolincarbonsäure,
. 1-Cyclopropyl-6,8-difluor-7-(3-ethylaminomethyl-3-methyl-1-azetidinyl)-1,4-dihydro-4-oxo-3-chinolincarbonsäure,
. 1-Cyclopropyl-6-fluor-7-(3-trifluoracetamidomethyl-3-methyl-1-azetidinyl)-1,4-dihydro-4-oxo-3-chinolincarbonsäure,
. 1-Cyclopropyl-6-fluor-7-(3-trifluoracetamidoethylaminomethyl-3-methyl-1-azetidinyl)-1,4-dihydro-4-oxo-3-chinolincarbonsäure,
. 1-Cyclopropyl-6-fluor-7-(3-aminomethyl-3-methyl-1-azetidinyl)-1,4-dihydro-4-oxo-3-chinolincarbonsäure,
. 1-Cyclopropyl-6-fluor-7-(3-ethylaminomethyl-3-methyl-1-azetidinyl)-1,4-dihydro-4-oxo-3-chinolincarbonsäure,
. 1-Cyclopropyl-6,8-difluor-7-(3-N,N-dimethylamino-3-methyl-1-azetidinyl)-1,4-dihydro-4-oxo-3-chinolincarbonsäure,
. 1-Cyclopropyl-6-fluor-7-(3-amino-3-methyl-1-azetidinyl)-1,4-dihydro-4-oxo-3-chinolincarbonsäure,
. 1-Cyclopropyl-6,8-difluor-7-(3-dimethylamino-1-azetidinyl)-1,4-dihydro-4-oxo-3-chinolincarbonsäure,
. 1-Cyclopropyl-6-fluor-7-(3-dimethylamino-1-azetidinyl)-1,4-dihydro-4-oxo-3-chinolincarbonsäure,
. 1-Cyclopropyl-6,8-difluor-7-(3-amino-3-methyl-1-azetidinyl)-1,4-dihydro-4-oxo-3-chinolincarbonsäure-Hydrochlorid,
. 1-Cyclopropyl-6,8-difluor-7-(3-amino-3-methyl-1-azetidinyl)-1,4-dihydro-4-oxo-3-chinolincarbonsäure-Natriumsalz,
. 1-Cyclopropyl-6,8-difluor-7-(3-methylamino-1-azetidinyl)-1,4-dihydro-4-oxo-3-chinolincarbonsäure,
. 1-Cyclopropyl-6-fluor-7-(3-methylamino-1-azetidinyl)-1,4-dihydro-4-oxo-3-chinolincarbonsäure,
. 1-Cyclopropyl-6-fluor-7-(3-amino-1-azetidinyl)-1,4-dihydro-4-oxo-3-chinolincarbonsäure.

**6.** Verfahren zur Herstellung der Derivate der Formel I gemäß den Ansprüchen 1 bis 5, dadurch gekennzeichnet, daß es darin besteht, eine heterocyclische Verbindung der Formel II

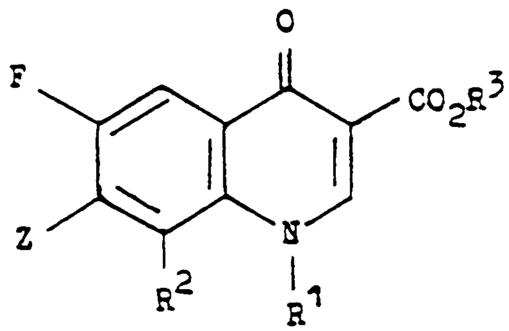

(II)

in der $R^1$, $R^2$ und $R^3$ die vorstehend angegebenen Bedeutungen besitzen und Z ein Halogenatom darstellt, mit einer Verbindung der Formel III

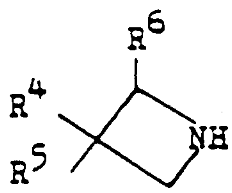

(III)

zur Reaktion zu bringen, in der $R^4$, $R^5$ und $R^6$ die vorstehend angegebenen Bedeutungen besitzen.

**7.** Verfahren zur Herstellung der Derivate der Formel I gemäß den Ansprüchen 1 bis 5, dadurch gekennzeichnet, daß es darin besteht, eine heterocyclische Verbindung der Formel II, in der $R^1$, $R^2$ und $R^3$ die vorstehend angegebenen Bedeutungen besitzen und Z ein Amino-Radikal darstellt, mit einer Verbindung der Formel IV

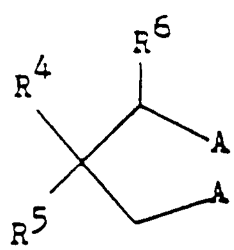

(IV)

zur Reaktion zu bringen, in der $R^4$, $R^5$ und $R^6$ die vorstehend angegebenen Bedeutungen besitzen und A ein Halogenatom, ein Hydroxyl-Radikal, ein niederes Alkylsulfonyloxy-Radikal mit 1 bis 4 Kohlenstoffatomen oder ein Phenyl-sulfonyloxy-Radikal bedeutet.

**8.** Als Medikamente die Verbindungen der allgemeinen Formel I und ihre therapeutisch akzeptablen Salze gemäß einem der Ansprüche 1 bis 5, speziell als antibakterielle Mittel.

**9.** Pharmazeutische Zusammensetzungen, dadurch gekennzeichnet, daß sie außer einem pharmazeutisch akzeptablen Träger mindestens eine Verbindung der allgemeinen Formel I oder eines ihrer pharmazeu-tisch akzeptablen Salze, gemäß einem der Ansprüche 1 bis 5, enthalten.